# EUROPEAN PATENT SPECIFICATION

(11) **EP 3 368 073 B1**
(45) Date of publication and mention of the grant of the patent: **15.05.2024**
(21) Application number: 16791271.6
(22) Date of filing: 28.10.2016
(51) Int. Cl.: A61K 39/395, A61K 39/00, C07K 16/18, C07K 16/40

(54) **A METHOD OF INHIBITING EXACERBATIONS OF T CELL-MEDIATED ALLOGRAFT VASCULOPATHY**
VERFAHREN ZUR HEMMUNG VON VERSCHLECHTERUNGEN DER T-ZELL-VERMITTELTEN ALLOTRANSPLANTAT-VASKULOPATHIE
PROCÉDÉ D'INHIBITION D'EXACERBATIONS DE VASCULOPATHIES D'ALLOGREFFE MÉDIÉES PAR LES CELLULES T

(30) Priority: 30.10.2015 US 201562248873 P; 12.04.2016 US 201662321632 P
(43) Date of publication of application: 05.09.2018
(73) Proprietor: Alexion Pharmaceuticals, Inc., New Haven, CT 06510 (US); Yale University, New Haven, CT 06510 (US)
(72) Inventor: POBER, Jordan, Guilford, Connecticut 06437 (US); JANE-WIT, Daniel, Branford, Connecticut 06405 (US); QIN, Lingfeng, Hamden, Connecticut 06518 (US); WANG, Yi, Woodbridge, Connecticut 06525 (US)
(74) Representative: J A Kemp LLP
(86) International application number: PCT/US2016/059279
(87) International publication number: WO 2017/075325

(56) References cited:
- WO-A1-2010/054403
- WO-A1-2013/134516
- WO-A1-2015/023972
- WO-A1-2015/134894
- L. QIN ET AL: "Complement C5 Inhibition Reduces T Cell-Mediated Allograft Vasculopathy Caused by Both Alloantibody and Ischemia Reperfusion Injury in Humanized Mice", AMERICAN JOURNAL OF TRANSPLANTATION, vol. 16, no. 10, 14 June 2016 (2016-06-14) , pages 2865-2876, XP055336230, DK ISSN: 1600-6135, DOI: 10.1111/ajt.13834

## Description

### SEQUENCE LISTING

The instant application contains a Sequence Listing which has been submitted electronically in ASCII format and is hereby incorporated by reference in its entirety. Said ASCII copy, created on October 28, 2016, is named ALXN345PCT_SL.txt and is 40,638 bytes in size.

### TECHNICAL FIELD

This invention relates to the fields of solid organ transplants and more specifically to methods of treating and preventing allograft vasculopathy. The invention is defined by the claims and any other aspects, configurations or embodiments set forth herein not falling within the scope of the claims are for information only.
Any references in the description to methods of treatment refer to the compounds, pharmaceutical compositions and medicaments of the present invention for use in a method for treatment of the human (or animal) body by therapy (or for diagnosis).

### BACKGROUND

Longevity of solid organ allografts is frequently limited by allograft vasculopathy (AV), a condition where irreversible stenoses form throughout graft vessels. AV lesions have been observed in cardiac (Beygui F et al. J Heart Lung Transplant 2010; 29: 316-322; Lee MS et al. Cardiac allograft vasculopathy Rev Cardiovasc Med 2011; 12: 143-152; Costello JP et al. Tex Heart Inst J 2013; 40: 395-399; Hollis IB et al. Pharmacotherapy 2015; 35: 489-501), renal (Bagnasco SM, Kraus ES, Curr Opin Organ Transplant 2015; 20: 343-347; Gupta G et al. Transplantation 2014; 97: 1240-1246,) and composite allografts (Mundinger GS, Drachenberg CB. Curr Opin Organ Transplant 2014; 19: 309-14; Unadkat JV et al. Am J Transplant 2009; 10: 251-261; Unadkat JV et al. Transplant Proc 2009; 41: 542-545), where they substantially contribute to late graft loss. AV lesions occur in two distinct forms. In the first and most well-characterized form, affected vessels contain a diffusely expanded neointima made up of smooth muscle-like cells along with sub-endothelial infiltrates of T cells and macrophages. Pober JS et al. Arterioscler Thromb Vase Biol. 2014; 34: 1609-1614. Similar appearing neointimal lesions can be induced in human artery segments in response to human IFN-γ (Tellides G et al. Nature 2000; 403: 207-11) or IFN-γ-producing human T cells (Shiao SL et al, J Immunol 2007; 179: 4397-404). In a more recently appreciated form of AV, luminal endothelial cells (ECs) locally activate the coagulation cascade (Jane-Wit D et al. Circulation 2013; 128: 2504-2516), resulting in occlusive thromboses (Yi T, et al. Arterioscler Thromb Vase Biol 2012; 32: 353-360; Jane-Wit D et al. Proc Natl Acad Sci U S A. 2015; 112: 9686-9691). Thrombotic AV lesions affecting microvessels and large-caliber vessels (Miwa T et al. J Immunol 2013; 190: 3552-3559) are associated with worsened long-term graft outcomes (Elvington A et al. J Immunol 2012; 189: 4640-4647). WO 2010/054403 A1 mentions using an anti-C5 antibody in a method for allogenic organ or tissue transplantation to substantially inhibit systemic complement activity in the patient but does not provide any results of actual treatment nor provide information about a possible beneficial effect in kidney transplant patients.

There are no approved therapeutic agents indicated for the treatment or prevention of AV.

### SUMMARY

This disclosure provides a method of reducing the likelihood of forming a T cell-mediated allograft vasculopathy lesion in a mammalian transplant recipient comprising transplanting an allograft from a donor to the recipient and administering a therapeutically effective amount of an anti-C5 antibody, or antigen-binding fragment thereof, to the recipient; the anti-C5 antibody, or antigen-binding fragment thereof, reduces the likelihood of forming an allograft vasculopathy lesion in the allograft, compared to the absence of treatment with an anti-C5 antibody, or antigen-binding fragment thereof.

This disclosure also provides a method of reducing the likelihood of forming a T cell-mediated allograft vasculopathy lesion in a mammalian transplant recipient that includes the steps of: selecting a mammalian allograft donor and an allograft recipient; transplanting an allograft from the donor to the recipient; and, administering a therapeutically effective amount of an anti-C5 antibody, or antigen-binding fragment thereof, to the recipient; wherein the anti-C5 antibody, or antigen-binding fragment thereof, reduces the likelihood of forming an allograft vasculopathy lesion in the allograft, compared to the absence of treatment with an anti-C5 antibody, or antigen-binding fragment thereof.

In another embodiment, a method is provided of reducing the likelihood of forming a T cell-mediated allograft vasculopathy lesion in a mammalian transplant recipient that includes a step of pre-treating the allograft with the anti-C5 antibody, or antigen-binding fragment thereof, prior to the step of transplanting the allograft.

In another embodiment, treating the recipient with the anti-C5 antibody, or antigen-binding fragment thereof, and/or treating the allograft with the anti-C5 antibody, or antigen-binding fragment thereof, reduces the likelihood of an ischemia reperfusion (IR) injury to the allograft, compared to the absence of treatment with an anti-C5 antibody, or antigen-binding fragment thereof.

In another embodiment, treating the recipient with the anti-C5 antibody, or antigen-binding fragment thereof, and/or treating of the allograft with the anti-C5 antibody, or antigen-binding fragment thereof, reduces the likelihood of a donor specific antibody (DSA) induced allograft injury, compared to the absence of treatment with an anti-C5 antibody, or antigen-binding fragment thereof.

In another embodiment, treating the recipient with the anti-C5 antibody, or antigen-binding fragment thereof, and/or treating the allograft with the anti-C5 antibody, or antigen-binding fragment thereof, reduces the likelihood of thrombotic complications of transplant-associated IR injury, compared to the absence of treatment with an anti-C5 antibody, or antigen-binding fragment thereof.

In another embodiment, treating the recipient with the anti-C5 antibody, or antigen-binding fragment thereof, and/or treating the allograft with the anti-C5 antibody, or antigen-binding fragment thereof, reduces the likelihood of IR injury induced membrane attack complex assembly in microvessels and large-caliber vessels of the allograft, compared to the absence of treatment with an anti-C5 antibody, or antigen-binding fragment thereof.

In another embodiment, treating the recipient with the anti-C5 antibody, or antigen-binding fragment thereof, and/or treating of the allograft with the anti-C5 antibody, or antigen-binding fragment thereof, reduces the likelihood of IR induced activation of non-canonical NF-κB signaling in the allograft, compared to the absence of treatment with an anti-C5 antibody, or antigen-binding fragment thereof.

In another embodiment, treating the recipient with the anti-C5 antibody, or antigen-binding fragment thereof, and/or treating the allograft with the anti-C5 antibody, or antigen-binding fragment thereof, reduces the likelihood of IR injury induced NIK expression, compared to the absence of treatment with an anti-C5 antibody, or antigen-binding fragment thereof.

In another embodiment, wherein treating the recipient and/or treating of the allograft with the anti-C5 antibody, or antigen-binding fragment thereof, reduces the likelihood of forming a diffusely expanded neointima made up of smooth muscle-like cells along with sub-endothelial infiltrates of T cells and macrophages in a vessel of the allograft, compared to the absence of treatment with an anti-C5 antibody, or antigen-binding fragment thereof.

In another embodiment, treating the recipient with the anti-C5 antibody, or antigen-binding fragment thereof, and/or treating the allograft with the anti-C5 antibody, or antigen-binding fragment thereof, reduces the likelihood of forming an occlusive thromboses, in a vascular vessel of the allograft, compared to the absence of treatment with an anti-C5 antibody, or antigen-binding fragment thereof.

In another embodiment, the disclosure provides a method of reducing the likelihood of forming a T cell-mediated allograft vasculopathy lesion in a mammalian transplant recipient that includes a step of administering to the recipient an anti-meningococcal vaccine and/or antibiotics prior to administering the anti-C5 antibody, or antigen-binding fragment thereof.

In another embodiment, the disclosure provides a method of reducing the likelihood of forming a T cell-mediated allograft vasculopathy lesion in a mammalian transplant recipient that includes a step of administering to the recipient an immunosuppressive therapy, wherein the immunosuppressive therapy is selected from the group consisting of corticosteroids, azathioprine (AZA), mycophenolate mofetil (MMF), methotrexate, tacrolimus, cyclosporine or cyclophosphamide either in combination or as a mono-therapy.

In another embodiment, the anti-C5 antibody, or antigen-binding fragment thereof, is administered intravenously.

In another embodiment, the mammalian transplant recipient is a human recipient.

In another embodiment, the anti-C5 antibody, or antigen-binding fragment thereof is eculizumab.

In another embodiment, the anti-C5 antibody, or antigen-binding fragment thereof is BNJ441.

In another embodiment, the anti-C5 antibody, or antigen-binding fragment thereof is BNJ421.

In another embodiment, the anti-C5 antibody, or antigen-binding fragment thereof, comprises CDR1, CDR2, and CDR3 heavy chain sequences as set forth in SEQ ID NOs: 1, 2, and 3, respectively, and CDR1, CDR2, and CDR3 light chain sequences as set forth in SEQ ID NOs: 4, 5, and 6, respectively.

In another embodiment, the anti-C5 antibody, or antigen-binding fragment thereof, comprises the VH domain having the sequence set forth in SEQ ID NO:7, and the VL domain having the sequence set forth in SEQ ID NO:8, respectively.

In another embodiment, the anti-C5 antibody, or antigen-binding fragment thereof, comprises a heavy chain constant region having the amino acid sequences set forth in SEQ ID NO: 9.

In another embodiment, the anti-C5 antibody, or antigen-binding fragment thereof, comprises the entire heavy chain and light chains having the amino acid sequences set forth in SEQ ID NO: 10 and SEQ ID NO: 11, respectively.

In another embodiment, the anti-C5 antibody, or antigen-binding fragment thereof, comprises CDR1, CDR2, and CDR3 heavy chain sequences as set forth in SEQ ID NOs:19, 18, and 3, respectively, and CDR1, CDR2, and CDR3 light chain sequences as set forth in SEQ ID NOs: 4, 5, and 6, respectively.

In another embodiment, the anti-C5 antibody, or antigen-binding fragment thereof comprises the VH domain having the sequence set forth in SEQ ID NO:12, and the VL domain having the sequence set forth in SEQ ID NO:8, respectively.

In another embodiment, the anti-C5 antibody, or antigen-binding fragment thereof comprises a heavy chain constant region having the amino acid sequences set forth in SEQ ID NO: 13.

In another embodiment, the anti-C5 antibody, or antigen-binding fragment thereof comprises the entire heavy chain and light chains having the amino acid sequences set forth in SEQ ID NO: 14 and SEQ ID NO: 11, respectively.

In another embodiment, the anti-C5 antibody, or antigen-binding fragment thereof comprises the entire heavy chain and light chains having the amino acid sequences set forth in SEQ ID NO: 20 and SEQ ID NO: 11, respectively.

In another embodiment, the anti-C5 antibody, or antigen-binding fragment thereof comprises CDR1, CDR2, and CDR3 heavy chain sequences as set forth in SEQ ID NOs:21, 22, and 23, respectively, and CDR1, CDR2, and CDR3 light chain sequences as set forth in SEQ ID NOs: 24, 25, and 26, respectively.

In another embodiment, the anti-C5 antibody, or antigen-binding fragment thereof comprises the VH domain having the sequence set forth in SEQ ID NO:27, and the VL domain having the sequence set forth in SEQ ID NO:28.

In another embodiment, the anti-C5 antibody, or antigen-binding fragment thereof comprises CDR1, CDR2, and CDR3 heavy chain sequences as set forth in SEQ ID NOs:29, 30, and 31, respectively, and CDR1, CDR2, and CDR3 light chain sequences as set forth in SEQ ID NOs: 32, 33, and 34, respectively.

In another embodiment, the disclosure provides that the anti-C5 antibody, or antigen-binding fragment thereof comprises the VH domain having the sequence set forth in SEQ ID NO:35, and the VL domain having the sequence set forth in SEQ ID NO:36, respectively.

In another embodiment, the disclosure provides a kit for preventing the formation of an allograft vasculopathy lesion in an allograft of a mammalian transplant recipient, the kit comprising: a dose of an anti-C5 antibody, or antigen-binding fragment thereof, wherein the anti-C5 antibody, or antigen-binding fragment thereof, comprises CDR1, CDR2, and CDR3 heavy chain sequences as set forth in SEQ ID NOs: 1, 2, and 3, respectively, and CDR1, CDR2, and CDR3 light chain sequences as set forth in SEQ ID NOs: 4, 5, and 6, respectively; instructions for using the anti-C5 antibody, or antigen-binding fragment thereof, in the method of reducing the likelihood of forming a T cell-mediated allograft vasculopathy lesion in a mammalian transplant recipient, and a syringe.

### Brief Description of the Drawings

**FIG. 1** demonstrates that anti-C5 mAb does not exert immunomodulatory effects on T cell-mediated AV in the absence of terminal complement activation.
**FIG. 2** demonstrates that blockade of MAC assembly and not C5a generation by anti-C5 mAb attenuates EC activation in response to DSA.
**FIG. 3** demonstrates that anti-C5 antibody reduces neointimal lesions following alloantibody-induced complement activation.
**FIG. 4** demonstrates that IRI activates complement and induces MAC formation in the artery wall without overt target cell loss.
**FIG. 5** demonstrates that terminal complement inhibition blocks IRI-induced MAC formation, non-canonical NF-κB, and EC Activation.
**FIG. 6** demonstrates the results of an experiment demonstrating that anti-C5 antibody attenuates neointimal and thrombotic AV lesions following IRI.
**FIG. 7** shows naive SCID/bg mice containing coronary artery interposition grafts implanted in the descending aorta received i.v. injection of PRA sera prior to graft harvest 18 hours later and analysis by I.F. (n=3 treatment pairs). C5a activity levels were assessed in murine plasma at the time of graft harvest as depicted in Fig 2g following treatment with blocking anti-C5a mAb CLS026 or an isotype control mAb MOPC1 (n=3 treatment pairs).
**FIG. 8** shows coronary artery grafts explanted, placed in organ culture under anoxic conditions for the times indicated and stained for hypoxyprobe (n=5 mice for each time point). Scale bars indicate 63µm.
**FIG. 9** shows coronary artery grafts explanted, placed in organ culture under anoxic conditions for 12 hours and then reimplanted into a second set of naive SCID/bg hosts. Grafts were harvested 18 hours after re-implantation and serial sections were taken for immunofluorescence analysis adjacent to the distal suture line, indicating the margin between human arterial graft and murine descending aorta tissue.

### DETAILED DESCRIPION

### Definitions

As used herein, the word "a" or "plurality" before a noun represents one or more of the particular noun. For example, the phrase "a mammalian cell" represents "one or more mammalian cells."

As used herein, the term "complement-mediated damage" refers to a pathological condition in which complement activation contributes in an observable or measurable way to the pathology of the condition. For example, complement-mediated damage can be characterized by the destruction of cells through complement activation.

As used herein, the term "reducing a risk" or "improving a likelihood" refers to a change in a rate of occurrence of an event by a statistically significant amount. For example, in one embodiment, reducing refers to either partially or completely preventing an event. In one embodiment, "reducing" means a decrease by at least 10% compared to a reference level, for example a decrease by at least about 15%, or at least about 20%, or at least about 25%, or at least about 30%, or at least about 35%, or at least about 40%, or at least about 45%, or at least about 50%, or at least about 55%, or at least about 60%, or at least about 65%, or at least about 70%, or at least about 75%, or at least about 80%, or at least about 85%, or at least about 90%, or at least about 95%, or up to and including a 100% decrease compared to a reference sample, or any decrease between 10-100% compared to a reference level.

As used herein, the term "vasculopathy" refers to any disease affecting blood vessels. The term "thrombosis" or "thrombotic" refers to a blood clot, or its formation, inside a blood vessel, obstructing the flow of blood through the circulatory system. The term "stenosis" or "stenotic" refers to a blood clot, or its formation, inside a blood vessel, obstructing the flow of blood through the circulatory system.

As used herein, the term "reducing the incidence" and "improving function" refer to a beneficial effect, e.g., amelioration or an improvement over baseline. Frequently the improvement over baseline is statistically significant. For example, "reducing the incidence" and "improving function" may refer to an amelioration of at least 10% as compared to a reference level, for example, an improvement of at least about 20%, or at least about 30%, or at least about 40%, or at least about 50%, or at least about 60%, or at least about 70%, or at least about 80%, or at least about 90% or up to and including a 100% improvement or any improvement between 10-100% as compared to a reference level, or at least about a 2-fold, or at least about a 3-fold, or at least about a 4-fold, or at least about a 5-fold, or at least about a 6-fold, or at least about a 7-fold, or at least about a 8-fold, or at least about a 9-fold, or at least about a 10-fold improvement, or any improvement between 2-fold and 10-fold or greater, as compared to a reference level.

As used herein, the term "transplant" refers to the replacement of an organ, for example, a kidney, in a human or non-human animal recipient. The purpose of replacement is to remove a diseased organ or tissue in the host and replace it with a healthy organ or tissue from the donor. Where the donor and the recipient are the same species the transplant is known as an "allograft". Where the donor and the recipient are dissimilar species the transplant is known as a "xenograft". The techniques necessary for transplantation are varied and depend to a large extent on the nature of the organ being transplanted. The success of the transplant as a therapeutic modality depends on a number of possible physiological outcomes. For example, the host may reject the new organ via antibody-dependent hyperacute rejection mechanisms, cell-mediated acute rejection, or chronic degenerative processes.

As used herein, the term "reperfusion" refers to the act of restoring the flow of blood to an organ or tissue (e.g., a kidney). Reperfusion injury or ischemic reperfusion injury ("IR injury") is the tissue damage caused when blood supply returns to the tissue after a period of ischemia or lack of oxygen. The absence of oxygen and nutrients from blood during the ischemic period creates a condition in which the restoration of circulation results in inflammation and oxidative damage through the induction of oxidative stress rather than restoration of normal function. Kidneys from deceased donors are exposed to much greater ischemic damage, as compared to living donors, before recovery and show reduced chances for proper early as well as long-term function. Kosieradzki M et al. Transplant Proc. 2008 Dec; 40(10):3279-88. Techniques for reperfusion of organs and tissue are known in the art, and are disclosed, for example, in International Patent Application WO2011/002926, and U.S. Pat. Nos. 5,723,282 and 5,699,793.

The term "sensitized" used in connection with a recipient refers to a recipient that has exceptionally high antibody levels that react to foreign tissue, such as a donated organ.

As used here the term "rejection" refers to the process or processes by which the immune response of an organ transplant recipient mounts a reaction against the transplanted organ, cell or tissue, sufficient to impair or destroy normal function of the organ. The immune system response can involve specific (antibody and T cell-dependent) or non-specific (phagocytic, complement-dependent, etc.) mechanisms, or both.

The term "effective amount" refers to an amount of an agent that provides the desired biological, therapeutic, and/or prophylactic result. That result can be reduction, amelioration, palliation, lessening, delaying, and/or alleviation of one or more of the signs or symptoms of IR injury or any other desired alteration of a biological system.

The term "antibody" is known in the art. Briefly, it can refer to a whole antibody comprising two light chain polypeptides and two heavy chain polypeptides. Whole antibodies include different antibody isotypes, including IgM, IgG, IgA, IgD, and IgE antibodies. The term "antibody" includes, for example, a polyclonal antibody, a monoclonal antibody, a chimerized or chimeric antibody, a humanized antibody, a primatized antibody, a deimmunized antibody, and a fully human antibody. The antibody can be made in or derived from any of a variety of species, e.g., mammals such as humans, non-human primates (e.g., orangutan, baboons, or chimpanzees), horses, llamas, cattle, pigs, sheep, goats, dogs, cats, rabbits, guinea pigs, gerbils, hamsters, rats, and mice. The antibody can be a purified and/or a recombinant antibody.

The term "donor" refers to a deceased individual who has irreversibly lost all brain function. This may occur after an injury such as a fall, motor vehicle accident or a stroke. The determination of irreversibility, as well as the determination that all brain function is not present, are only made after repeated, confirmatory testing over a prolonged period of time.

The term "donor specific antibody", DSA or "alloantibody" refers to antibodies that are anti-HLA antibodies, specifically generated against donor cells.

The term "ischemia reperfusion injury" refers to the tissue damage caused when blood supply returns to the tissue after a period of ischemia or lack of oxygen (anoxia, hypoxia).

For the terms "for example" and "such as," and grammatical equivalences thereof, the phrase "and without limitation" is understood to follow unless explicitly stated otherwise. As used herein, the term "about" is meant to account for variations due to experimental error. All measurements reported herein are understood to be modified by the term "about," whether or not the term is explicitly used, unless explicitly stated otherwise. As used herein, the singular forms "a," "an," and "the" include plural referents unless the context clearly dictates otherwise.

Unless otherwise defined, all technical and scientific terms used herein have the same meaning as commonly understood by one of ordinary skill in the art to which this invention belongs. Methods and materials are described herein for use in the present invention; other, suitable methods and materials known in the art can also be used. The materials, methods, and examples are illustrative only and not intended to be limiting. All publications, patent applications, patents, sequences, database entries, and other references mentioned herein are incorporated by reference in their entirety. In case of conflict, the present specification, including definitions, will control.

### The Complement System

The complement system acts in conjunction with other immunological systems of the body to defend against intrusion of cellular and viral pathogens. There are at least 25 complement proteins, which are found as a complex collection of plasma proteins and membrane cofactors. The plasma proteins make up about 10% of the globulins in vertebrate serum. Complement components achieve their immune defensive functions by interacting in a series of intricate but precise enzymatic cleavage and membrane binding events. The resulting complement cascade leads to the production of products with opsonic, immunoregulatory, and lytic functions. A concise summary of the biologic activities associated with complement activation is provided, for example, in The Merck Manual, 16th Edition.

The complement cascade can progress via the classical pathway (CP), the lectin pathway, or the alternative pathway (AP). The lectin pathway is typically initiated with binding of mannose-binding lectin (MBL) to high mannose substrates. The AP can be antibody independent, and can be initiated by certain molecules on pathogen surfaces. The CP is typically initiated by antibody recognition of, and binding to, an antigenic site on a target cell. These pathways converge at the C3 convertase--the point where complement component C3 is cleaved by an active protease to yield C3a and C3b.

The AP C3 convertase is initiated by the spontaneous hydrolysis of complement component C3, which is abundant in the plasma fraction of blood. This process, also known as "tickover," occurs through the spontaneous cleavage of a thioester bond in C3 to form C3i or C3(H₂O) . Tickover is facilitated by the presence of surfaces that support the binding of activated C3 and/or have neutral or positive charge characteristics (e.g., bacterial cell surfaces). This formation of C3(H₂O) allows for the binding of plasma protein Factor B, which in turn allows Factor D to cleave Factor B into Ba and Bb. The Bb fragment remains bound to C3 to form a complex containing C3(H₂O)Bb--the "fluid-phase" or "initiation" C3 convertase. Although only produced in small amounts, the fluid-phase C3 convertase can cleave multiple C3 proteins into C3a and C3b and results in the generation of C3b and its subsequent covalent binding to a surface (e.g., a bacterial surface). Factor B bound to the surface-bound C3b is cleaved by Factor D to thus form the surface-bound AP C3 convertase complex containing C3b,Bb. (See, e.g., Muller-Eberhard (1988) Ann Rev Biochem 57:321-347.)

The AP C5 convertase--(C3b) ₂, Bb--is formed upon addition of a second C3b monomer to the AP C3 convertase. (See, e.g., Medicus et al. (1976) J Exp Med 144:1076-1093 and Fearon et al. (1975) J Exp Med 142:856-863.) The role of the second C3b molecule is to bind C5 and present it for cleavage by Bb. (See, e.g., Isenman et al. (1980) J Immunol 124:326-331.) The AP C3 and C5 convertases are stabilized by the addition of the trimeric protein properdin as described in, e.g., Medicus et al. (1976), *supra.* However, properdin binding is not required to form a functioning alternative pathway C3 or C5 convertase. (See, e.g., Schreiber et al. (1978) Proc Natl Acad Sci USA 75: 3948-3952 and Sissons et al. (1980) Proc Natl Acad Sci USA 77: 559-562) .

The CP C3 convertase is formed upon interaction of complement component C1, which is a complex of C1q, C1r, and C1s, with an antibody that is bound to a target antigen (e.g., a microbial antigen). The binding of the C1q portion of C1 to the antibody-antigen complex causes a conformational change in C1 that activates C1r. Active C1r then cleaves the C1-associated C1s to thereby generate an active serine protease. Active C1s cleaves complement component C4 into C4b and C4a. Like C3b, the newly generated C4b fragment contains a highly reactive thiol that readily forms amide or ester bonds with suitable molecules on a target surface (e.g., a microbial cell surface). C1s also cleaves complement component C2 into C2b and C2a. The complex formed by C4b and C2a is the CP C3 convertase, which is capable of processing C3 into C3a and C3b. The CP C5 convertase--C4b, C2a, C3b--is formed upon addition of a C3b monomer to the CP C3 convertase. (See, e.g., Muller-Eberhard (1988), *supra* and Cooper et al. (1970) J Exp Med 132:775-793.)

In addition to its role in C3 and C5 convertases, C3b also functions as an opsonin through its interaction with complement receptors present on the surfaces of antigen-presenting cells such as macrophages and dendritic cells. The opsonic function of C3b is generally considered to be one of the most important anti-infective functions of the complement system. Patients with genetic lesions that block C3b function are prone to infection by a broad variety of pathogenic organisms, while patients with lesions later in the complement cascade sequence, i.e., patients with lesions that block C5 functions, are found to be more prone only to Neisseria infection, and then only somewhat more prone.

The AP and CP C5 convertases cleave C5 into C5a and C5b. Cleavage of C5 releases C5a, a potent anaphylatoxin and chemotactic factor, and C5b, which allows for the formation of the lytic terminal complement complex, C5b-9. C5b combines with C6, C7, and C8 to form the C5b-8 complex at the surface of the target cell. Upon binding of several C9 molecules, the membrane attack complex (MAC, C5b-9, terminal complement complex--TCC) is formed. When sufficient numbers of MACs insert into target cell membranes the openings they create (MAC pores) mediate rapid osmotic lysis of the target cells.

### Anti-C5 Antibodies

The anti-C5 antibodies described herein bind to complement component C5 (e.g., human C5) and inhibit the cleavage of C5 into fragments C5a and C5b. Anti-C5 antibodies (or V_{H}/V_{L} domains derived therefrom) suitable for use in the invention can be generated using methods well known in the art. Alternatively, art recognized anti-C5 antibodies can be used. Antibodies that compete with any of these art-recognized antibodies for binding to C5 also can be used.

An exemplary anti-C5 antibody is eculizumab comprising heavy and light chains having the sequences shown in SEQ ID NOs: 10 and 11, respectively, or antigen binding fragments and variants thereof. Eculizumab (also known as Soliris^{®}) is described in US Patent No: 6,355,245. Eculizumab is a humanized monoclonal antibody that is a terminal complement inhibitor.

In other embodiments, the antibody comprises the heavy and light chain CDRs or variable regions of eculizumab. Accordingly, in one embodiment, the antibody comprises the CDR1, CDR2, and CDR3 domains of the VH region of eculizumab having the sequence set forth in SEQ ID NO: 7, and the CDR1, CDR2 and CDR3 domains of the VL region of eculizumab having the sequence set forth in SEQ ID NO: 8. In another embodiment, the antibody comprises heavy chain CDR1, CDR2 and CDR3 domains having the sequences set forth in SEQ ID NOs: 1, 2, and 3, respectively, and light chain CDR1, CDR2 and CDR3 domains having the sequences set forth in SEQ ID NOs: 4, 5, and 6, respectively. In another embodiment, the antibody comprises VH and VL regions having the amino acid sequences set forth in SEQ ID NO: 7 and SEQ ID NO: 8, respectively.

Another exemplary anti-C5 antibody is antibody BNJ441 comprising heavy and light chains having the sequences shown in SEQ ID NOs:14 and 11, respectively, or antigen binding fragments and variants thereof. BNJ441 (also known as ALXN1210) is described in PCT/US2015/019225 and US Patent No. 9,079,949. BNJ441 is a humanized monoclonal antibody that is structurally related to eculizumab (Soliris^{®}) . BNJ441 selectively binds to human complement protein C5, inhibiting its cleavage to C5a and C5b during complement activation. This inhibition prevents the release of the proinflammatory mediator C5a and the formation of the cytolytic pore-forming membrane attack complex C5b-9 while preserving the proximal or early components of complement activation *(e.g.,* C3 and C3b) essential for the opsonization of microorganisms and clearance of immune complexes.

In other embodiments, the antibody comprises the heavy and light chain CDRs or variable regions of BNJ441. Accordingly, in one embodiment, the antibody comprises the CDR1, CDR2, and CDR3 domains of the VH region of BNJ441 having the sequence set forth in SEQ ID NO:12, and the CDR1, CDR2 and CDR3 domains of the VL region of BNJ441 having the sequence set forth in SEQ ID NO:8. In another embodiment, the antibody comprises heavy chain CDR1, CDR2 and CDR3 domains having the sequences set forth in SEQ ID NOs:19, 18, and 3, respectively, and light chain CDR1, CDR2 and CDR3 domains having the sequences set forth in SEQ ID NOs:4, 5, and 6, respectively. In another embodiment, the antibody comprises VH and VL regions having the amino acid sequences set forth in SEQ ID NO:12 and SEQ ID NO:8, respectively. In another embodiment, the antibody may comprise the heavy chain constant region of BNJ441 having the amino acid sequences set forth in SEQ ID NO: 13.

Another exemplary anti-C5 antibody is antibody BNJ421 comprising heavy and light chains having the sequences shown in SEQ ID NOs:20 and 11, respectively, or antigen binding fragments and variants thereof. BNJ421 (also known as ALXN1211) is described in PCT/US2015/019225 and US Patent No.9,079,949,

In other embodiments, the antibody comprises the heavy and light chain CDRs or variable regions of BNJ421. Accordingly, in one embodiment, the antibody comprises the CDR1, CDR2, and CDR3 domains of the VH region of BNJ421 having the sequence set forth in SEQ ID NO:12, and the CDR1, CDR2 and CDR3 domains of the VL region of BNJ421 having the sequence set forth in SEQ ID NO:8. In another embodiment, the antibody comprises heavy chain CDR1, CDR2 and CDR3 domains having the sequences set forth in SEQ ID NOs:19, 18, and 3, respectively, and light chain CDR1, CDR2 and CDR3 domains having the sequences set forth in SEQ ID NOs:4, 5, and 6, respectively. In another embodiment, the antibody comprises VH and VL regions having the amino acid sequences set forth in SEQ ID NO:12 and SEQ ID NO:8, respectively. In another embodiment, the antibody may comprise the heavy chain constant region of BNJ421 having the amino acid sequences set forth in SEQ ID NO: 9.

Another exemplary anti-C5 antibody is the 7086 antibody described in US Patent Nos. 8,241,628 and 8,883,158. In one embodiments, the antibody may comprise the heavy and light chain CDRs or variable regions of the 7086 antibody. See US Patent Nos. 8,241,628 and 8,883,158. In another embodiment, the antibody, or a fragment thereof, comprises heavy chain CDR1, CDR2 and CDR3 domains having the sequences set forth in SEQ ID NOs:21, 22, and 23, respectively, and light chain CDR1, CDR2 and CDR3 domains having the sequences set forth in SEQ ID NOs:24, 25, and 26, respectively. In another embodiment, the antibody or antigen-binding fragment thereof may comprise the VH region of the 7086 antibody having the sequence set forth in SEQ ID NO:27, and the VL region of the 7086 antibody having the sequence set forth in SEQ ID NO:28.

Another exemplary anti-C5 antibody is the 8110 antibody also described in US Patent Nos. 8,241,628 and 8,883,158. In one embodiments, the antibody comprises the heavy and light chain CDRs or variable regions of the 8110 antibody. The antibody, or antigen-binding fragment thereof may comprise heavy chain CDR1, CDR2 and CDR3 domains having the sequences set forth in SEQ ID NOs:29, 30, and 31, respectively, and light chain CDR1, CDR2 and CDR3 domains having the sequences set forth in SEQ ID NOs:32, 33, and 34, respectively. In another embodiment, the antibody comprises the VH region of the 8110 antibody having the sequence set forth in SEQ ID NO:35, and the VL region of the 8110 antibody having the sequence set forth in SEQ ID NO:36.

The exact boundaries of CDRs have been defined differently according to different methods. In some embodiments, the positions of the CDRs or framework regions within a light or heavy chain variable domain can be as defined by Kabat et al. [(1991) "Sequences of Proteins of Immunological Interest." NIH Publication No. 91-3242, U.S. Department of Health and Human Services, Bethesda, MD]. In such cases, the CDRs can be referred to as "Kabat CDRs" (e.g., "Kabat LCDR2" or "Kabat HCDR1"). In some embodiments, the positions of the CDRs of a light or heavy chain variable region can be as defined by Chothia et al. (1989) Nature 342:877-883. Accordingly, these regions can be referred to as "Chothia CDRs" (e.g., "Chothia LCDR2" or "Chothia HCDR3"). In some embodiments, the positions of the CDRs of the light and heavy chain variable regions can be as defined by a Kabat-Chothia combined definition. In such embodiments, these regions can be referred to as "combined Kabat-Chothia CDRs". Thomas et al. [(1996) Mol Immunol 33(17/18):1389-1401] exemplifies the identification of CDR boundaries according to Kabat and Chothia definitions.

Methods for determining whether an antibody binds to a protein antigen and/or the affinity for an antibody to a protein antigen are known in the art. For example, the binding of an antibody to a protein antigen can be detected and/or quantified using a variety of techniques such as, but not limited to, Western blot, dot blot, surface plasmon resonance (SPR) method (e.g., BIAcore system; Pharmacia Biosensor AB, Uppsala, Sweden and Piscataway, N.J.), or enzyme-linked immunosorbent assay (ELISA). See, *e.g.,* Benny K. C. Lo (2004) "Antibody Engineering: Methods and Protocols," Humana Press (ISBN: 1588290921); Johne et al. (1993) J Immunol Meth 160:191-198; Jonsson et al. (1993) Ann Biol Clin 51:19-26; and Jonsson et al. (1991) Biotechniques 11:620-627.

In one embodiment, the antibody competes for binding with, and/or binds to the same epitope on C5 as, the antibodies described herein. The term "binds to the same epitope" with reference to two or more antibodies means that the antibodies bind to the same segment of amino acid residues, as determined by a given method. Techniques for determining whether antibodies bind to the "same epitope on C5" with the antibodies described herein include, for example, epitope mapping methods, such as, x-ray analyses of crystals of antigen:antibody complexes which provides atomic resolution of the epitope and hydrogen/deuterium exchange mass spectrometry (HDX-MS). Other methods monitor the binding of the antibody to peptide antigen fragments or mutated variations of the antigen where loss of binding due to a modification of an amino acid residue within the antigen sequence is often considered an indication of an epitope component. In addition, computational combinatorial methods for epitope mapping can also be used. These methods rely on the ability of the antibody of interest to affinity isolate specific short peptides from combinatorial phage display peptide libraries. Antibodies having the same VH and VL or the same CDR1, 2 and 3 sequences are expected to bind to the same epitope.

Antibodies that "compete with another antibody for binding to a target" refer to antibodies that inhibit (partially or completely) the binding of the other antibody to the target. Whether two antibodies compete with each other for binding to a target, *i.e.,* whether and to what extent one antibody inhibits the binding of the other antibody to a target, may be determined using known competition experiments. In certain embodiments, an antibody competes with, and inhibits binding of another antibody to a target by at least 10%, 20%, 30%, 40%, 50%, 60%, 70%, 80%, 90% or 100%. The level of inhibition or competition may be different depending on which antibody is the "blocking antibody" (i.e., the cold antibody that is incubated first with the target). Competing antibodies bind to the same epitope, an overlapping epitope or to adjacent epitopes (e.g., as evidenced by steric hindrance). Anti-C5 antibodies, or antigen-binding fragments thereof described herein, used in the methods described herein can be generated using a variety of art-recognized techniques.

Monoclonal antibodies may be obtained by various techniques familiar to those skilled in the art. Briefly, spleen cells from an animal immunized with a desired antigen are immortalized, commonly by fusion with a myeloma cell (see, Kohler & Milstein, Eur. J. Immunol. 6: 511-519 (1976)). Alternative methods of immortalization include transformation with Epstein Barr Virus, oncogenes, or retroviruses, or other methods well known in the art. Colonies arising from single immortalized cells are screened for production of antibodies of the desired specificity and affinity for the antigen, and yield of the monoclonal antibodies produced by such cells may be enhanced by various techniques, including injection into the peritoneal cavity of a vertebrate host. Alternatively, one may isolate DNA sequences which encode a monoclonal antibody or a binding fragment thereof by screening a DNA library from human B cells according to the general protocol outlined by Huse, et al., Science 246: 1275-1281 (1989).

### Compositions and Formulations

Also, provided herein for use in the disclosed methods are compositions comprising an anti-C5 antibody, or an antigen binding fragment thereof.

Generally, the anti C5-antibody can be formulated as a pharmaceutical composition. The compositions will generally include a pharmaceutically acceptable carrier. As used herein, a "pharmaceutically acceptable carrier" refers to, and includes, any and all solvents, dispersion media, coatings, antibacterial and antifungal agents, isotonic and absorption delaying agents, and the like that are physiologically compatible. The compositions can include a pharmaceutically acceptable salt, e.g., an acid addition salt or a base addition salt (see e.g., Berge et al. (1977) J. Pharm. Sci. 66:1-19).

The compositions can be formulated according to standard methods. Pharmaceutical formulation is a well-established art, and is further described in, e.g., Gennaro (2000) "Remington: The Science and Practice of Pharmacy," 20th Edition, Lippincott, Williams & Wilkins (ISBN: 0683306472); Ansel et al. (1999) "Pharmaceutical Dosage Forms and Drug Delivery Systems," 7th Edition, Lippincott Williams & Wilkins Publishers (ISBN: 0683305727); and Kibbe (2000) "Handbook of Pharmaceutical Excipients American Pharmaceutical Association," 3rd Edition (ISBN: 091733096X).

The compositions can be formulated as a pharmaceutical solution, e.g., for administration to a subject for the treatment or prevention of a complement-associated disorder. The pharmaceutical compositions will generally include a pharmaceutically acceptable carrier. As used herein, a "pharmaceutically acceptable carrier" refers to, and includes, any and all solvents, dispersion media, coatings, antibacterial and antifungal agents, isotonic and absorption delaying agents, and the like that are physiologically compatible. The compositions can include a pharmaceutically acceptable salt, e.g., an acid addition salt or a base addition salt, sugars, carbohydrates, polyols and/or tonicity modifiers.

In some embodiments, a composition can be formulated, for example, as a buffered solution at a suitable concentration and suitable for storage at 2-8°C (e.g., 4°C). In some embodiments, a composition can be formulated for storage at a temperature below 0°C (e.g., -20°C or -80°C). In some embodiments, the composition can be formulated for storage for up to 2 years (e.g., one month, two months, three months, four months, five months, six months, seven months, eight months, nine months, 10 months, 11 months, 1 year, 1½ years, or 2 years) at 2-8°C (e.g., 4°C). Thus, in some embodiments, the compositions described herein are stable in storage for at least 1 year at 2-8°C (e.g., 4°C).

The pharmaceutical compositions can be in a variety of forms. These forms include, e.g., liquid, semi-solid and solid dosage forms, such as liquid solutions (e.g., injectable and infusible solutions), dispersions or suspensions, tablets, pills, powders, liposomes and suppositories. The preferred form depends, in part, on the intended mode of administration and therapeutic application. For example, compositions containing a composition intended for systemic or local delivery can be in the form of injectable or infusible solutions. Accordingly, the compositions can be formulated for administration by a parenteral mode (e.g., intravenous, subcutaneous, intraperitoneal, or intramuscular injection). "Parenteral administration," "administered parenterally," and other grammatically equivalent phrases, as used herein, refer to modes of administration other than enteral and topical administration, usually by injection, and include, without limitation, intravenous, intranasal, intraocular, pulmonary, intramuscular, intraarterial, intrathecal, intracapsular, intraorbital, intracardiac, intradermal, intrapulmonary, intraperitoneal, transtracheal, subcutaneous, subcuticular, intraarticular, subcapsular, subarachnoid, intraspinal, epidural, intracerebral, intracranial, intracarotid, and intrasternal injection and infusion.

### Methods of Treatment

The disclosure provides a method of reducing the likelihood of forming a T cell-mediated allograft vasculopathy lesion in a mammalian transplant recipient comprising transplanting an allograft from a donor to a recipient and administering a therapeutically effective amount of an anti-C5 antibody, or antigen-binding fragment thereof to the recipient, wherein the anti-C5 antibody, or antigen-binding fragment thereof, reduces the likelihood of forming an allograft vasculopathy lesion in the allograft, compared to the absence of treatment with an anti-C5 antibody, or antigen-binding fragment thereof.

In another embodiment, the disclosure provides a method of reducing the likelihood of forming a T cell-mediated allograft vasculopathy lesion in a mammalian transplant recipient comprising the steps of: selecting a mammalian allograft donor and an allograft recipient; transplanting an allograft from the donor to the recipient; and, administering a therapeutically effective amount of an anti-C5 antibody, or antigen-binding fragment thereof to the recipient; wherein the anti-C5 antibody, or antigen-binding fragment thereof reduces the likelihood of forming an allograft vasculopathy lesion in the allograft, compared to the absence of treatment with an anti-C5 antibody, or antigen-binding fragment thereof.

The method includes treatment for instances where the allograft has been exposed to an IR injury. It also includes treatment for instances where the allograft has been subjected to a DSA injury.

In one embodiment, the method comprises a step of pre-treating the donor allograft with the anti-C5 antibody, or antigen-binding fragment thereof, prior to the step of transplanting the allograft. See WO2015/023972.

In one embodiment, the antibody, or antigen-binding fragment thereof, is administered to the organ prior to transplantation(e.g., after removal of the organ from a donor mammal and before transplant of the organ into a recipient mammal). In one embodiment, the anti-C5 antibody, or antigen binding fragment, is administered at an organ procurement center. In another embodiment, the anti-C5 antibody, or antigen binding fragment, is administered immediately prior to transplantation, e.g., in a "back table" procedure within hours or minutes prior to transplantation. In one embodiment, the anti-C5 antibody, or antigen binding fragment, is administered after harvest or removal from the donor mammal, but prior to preservation of the organ. In another embodiment, the antibody, or antigen binding fragment, is administered to the organ during preservation. In another embodiment, the anti-C5 antibody, or antigen binding fragment, is administered after preservation, but prior to transplantation.

The anti-C5 antibody, or antigen binding fragment, can be administered to the organ by any suitable technique. In one embodiment, the anti-C5 antibody, or antigen binding fragment, is administered to the organ by perfusing the organ with a solution containing the anti-C5 antibody, or antigen binding fragment. In another embodiment, the organ is bathed in a solution containing the anti-C5 antibody, or antigen binding fragment. In one embodiment, the organ is perfused with or soaked in a solution containing the anti-C5 antibody, or antigen binding fragment for 0.5 hours to 60 hours or for 1 hour to 30 hours (e.g., for 30 minutes, 35 minutes, 40 minutes, 45 minutes, 50 minutes, 55 minutes, 1 hour, 1.5 hours, 2 hours, 2.5 hours, 3 hours, 3.5 hours, 4 hours, 4.5 hours, 5 hours, 5.5 hours, 6 hours, 6.5 hours, 7 hours, 7.5 hours, 8 hours, 8.5 hours, 9 hours, 9.5 hours, 10 hours, 10.5 hours, 11 hours, 11.5 hours, 12 hours, 12.5 hours, 13 hours, 13.5 hours, 14 hours, 14.5 hours, 15 hours, 15.5 hours, 16 hours, 16.5 hours, 17 hours, 17.5 hours, 18 hours, 18.5 hours, 19 hours, 19.5 hours, 20 hours, 21 hours, 22 hours, 23 hours, 24 hours, 25 hours, 26 hours, 27 hours, 28 hours, 29 hours, or 30 hours). The administration can involve two or more perfusions or soakings.

In some embodiments, the anti-C5 antibody, or antigen binding fragment, in a solution is from about 10 µg to about 500 mg per liter, including for example any of about 10 µg to about 50 pg, about 50 µg to about 100 pg, about 100 µg to about 200 pg, about 200 µg to about 300 pg, about 300 µg to about 500 pg, about 500 µg to about 1 mg, about 1 mg to about 10 mg, about 10 mg to about 50 mg, about 50 mg to about 100 mg, about 100 mg to about 200 mg, about 200 mg to about 300 mg, about 300 mg to about 400 mg, or about 400 mg to about 500 mg per liter. In some embodiments, the anti-C5 antibody, or antigen binding fragment, is at a concentration of about 10,20,30,40,50,60,70,80,90, 100,110,120,130,140,150, 160,170,180,190,200,210,220,230, 240,250,260,270,280,290,300,350,400,450,500,550, 600,650,700, 750, 800,850,900,950, 1000, 1500,2000,2500,3000,3500,4000,4500, 5000,5500,6000,6500, 7000, 7500,8000,8500,9000,9500, 10000, 15000,20000,30000, 40000, 50000, 60000, 70000, 80000, 90000, 100000, or above, pg/mL. In some embodiments, the amount of the anti-C5 antibody, or antigen binding fragment is at a concentration of about 130 pg/mL.

In another embodiment the antibody, or antigen-binding fragment thereof, is administered to a mammalian recipient by IV infusion. Frequently the antibody, or antigen-binding fragment thereof, is administered to the recipient by IV infusion over about 25 to about 45 minutes.

In another embodiment, the dose of the anti-C5 antibody, or antigen-binding fragment thereof, is administered to the recipient as a flat-fixed dose that is fixed irrespective of the weight of the mammalian recipient. In certain embodiments, dosage regimens are adjusted to provide the optimum desired response (e.g., an effective response).

In another embodiment, the anti-C5 antibody, or binding fragment thereof can be administered in a milligram per kilogram (mg/kg) dose based on the weight of the recipient. While in no way intended to be limiting, exemplary dosage ranges include, e.g., 1-100 mg/kg, 0.5-50 mg/kg, 0.1-100 mg/kg, 0.5-25 mg/kg, 1-20 mg/kg, and 1-10 mg/kg, 1-100 mg/kg, 0.5-50 mg/kg, 0.1-100 mg/kg, 0.5-25 mg/kg, 1-20 mg/kg, and 1-10 mg/kg. Exemplary dosages of the anti-C5 antibody, or antigen-binding fragment thereof, include, without limitation, 0.1 mg/kg, 0.5 mg/kg, 1.0 mg/kg, 2.0 mg/kg, 4 mg/kg, and 8 mg/kg, 0.1 mg/kg, 0.5 mg/kg, 1.0 mg/kg, 2.0 mg/kg, 4 mg/kg, and 8 mg/kg.

Often, the method provides that the plasma levels of the anti-C5 antibody, or binding fragment thereof is at about 50 to about 100 µg/mL following administration to the recipient.

In certain embodiments, the anti-C5 antibody, or antigen-binding fragment thereof, is administered in a multiphase dosing regimen. Often the regimen comprises two or more doses. The first dose may be administered prior to transplantation. A second dose may be administered from about 0.5 to about 48 hours or longer following transplantation. Often, the second dose is administered about 12 to about 36 hours after transplantation. The second dose may be administered about 18 to about 24 hours after transplantation. In certain embodiments, a third dose of the anti-C5 antibody, or antigen-binding fragment thereof, may be administered following the second dose. The third dose may be administered from about 0.5 to about 48 hours or longer following after the second dose.

In certain embodiments the recipient may undergo plasmaphreses following the transplant. The plasmaphreses may occur within 1, 2, 3, 4, 5, 6, 7, 8, 9, 10, 11, 12, 13, 14, 15, 16, 17, 18, 19, 20, 25, or 30 days of the transplant. Often plasmaphereses is performed within about 5 to about 10 days following the transplant. Usually the plasmaphereses is performed within about 10 days following the transplant.

The anti-C5 antibodies, or antigen binding fragments thereof, can be administered to a mammalian recipient by any suitable means. In one embodiment, the antibodies are administered by intravenous administration such as by perfusion or injection.

The anti-C5 antibodies, or antigen binding fragments thereof, also may be administered with one or more additional medicaments or therapeutic agents useful in the treatment of DGF, the additional agent being selected by the skilled artisan for its intended purpose. For example, the additional agent can be a therapeutic agent art-recognized as being useful to treat the disease or condition being treated by the antibody provided herein. The combination can also include more than one additional agent, e.g., two or three additional agents.

The anti-C5 the antibody, or antigen-binding fragment thereof, in various embodiments may be administered with an agent that is a protein, a peptide, a carbohydrate, a drug, a small molecule, and a genetic material (e.g., DNA or RNA). In various embodiments, the agent may be one or more cholinesterase inhibitors, one or more corticosteroids and/or one or more immunosuppressive drugs (most commonly azathioprine [AZA], cyclosporin, and/or mycophenolate mofetil [MMF].

In addition, the recipient may be administered one or more suitable therapeutic agents, prior to administration of the anti-C5 antibodies, or antigen binding fragments thereof. For example, in one embodiment, the recipient may be administered an antimeningococcal vaccine prior to treatment with the anti-C5 antibody, or antigen-binding fragment thereof. In another embodiment, the patient may be administered one or more antibiotics prior to treatment with the anti-C5 antibody, or antigen-binding fragment thereof.

The method provides that the mammalian transplant recipient may be a human recipient.

### Outcomes

Generally, treatment with the anti-C5 antibody, or antigen-binding fragment thereof provides for reducing the likelihood or preventing the formation of an allograft vasculopathy lesion in an allograft that has been transplanted from a mammalian donor to a mammalian recipient.

In another embodiment treatment with the anti-C5 antibody, or antigen-binding fragment thereof provides for reducing the likelihood of an ischemia reperfusion (IR) injury to the allograft, compared to the absence of treatment with an anti-C5 antibody, or antigen-binding fragment thereof.

In another embodiment treatment with the anti-C5 antibody, or antigen-binding fragment thereof provides for reducing the likelihood of a donor specific antibody (DSA) induced allograft injury, compared to the absence of treatment with an anti-C5 antibody, or antigen-binding fragment thereof.

In another embodiment treatment with the anti-C5 antibody, or antigen-binding fragment thereof provides for reducing the likelihood of thrombotic complications of transplant-associated IR injury, compared to the absence of treatment with an anti-C5 antibody, or antigen-binding fragment thereof.

In another embodiment treatment with the anti-C5 antibody, or antigen-binding fragment thereof provides for reducing the likelihood of IR injury induced membrane attack complex assembly in microvessels and large-caliber vessels of the allograft, compared to the absence of treatment with an anti-C5 antibody, or antigen-binding fragment thereof.

In another embodiment treatment with the anti-C5 antibody, or antigen-binding fragment thereof provides for reducing the likelihood of IR induced activation of non-canonical NF-κB signaling in the allograft, compared to the absence of treatment with an anti-C5 antibody, or antigen-binding fragment thereof.

In another embodiment treatment with the anti-C5 antibody, or antigen-binding fragment thereof provides for reducing the likelihood of IR injury induced NIK expression, compared to the absence of treatment with an anti-C5 antibody, or antigen-binding fragment thereof.

In another embodiment treatment with the anti-C5 antibody, or antigen-binding fragment thereof provides for reducing the likelihood of forming a diffusely expanded neointima made up of smooth muscle-like cells along with sub-endothelial infiltrates of T cells and macrophages in a vascular vessel of the allograft, compared to the absence of treatment with an anti-C5 antibody, or antigen-binding fragment thereof.

In another embodiment treatment with the anti-C5 antibody, or antigen-binding fragment thereof provides for reducing the likelihood of forming an occlusive thromboses, in a vascular vessel of the allograft, compared to the absence of treatment with an anti-C5 antibody, or antigen-binding fragment thereof.

### Kits and Unit Dosage Forms

Also provided herein are kits which include a pharmaceutical composition containing an anti-C5 antibody, or antigen-binding fragment thereof, such as eculizumab, and a pharmaceutically-acceptable carrier, in a therapeutically effective amount adapted for use in the preceding methods. The kits optionally also can include instructions, e.g., comprising administration schedules, to allow a practitioner (e.g., a physician, nurse, or patient) to administer the composition contained therein to administer the composition to a patient having allograft vasculopathy symptoms. The kit also may include a syringe.

Optionally, the kits include multiple packages of the single-dose pharmaceutical compositions each containing an effective amount of the anti-C5 antibody, or antigen-binding fragment thereof, for a single administration in accordance with the methods provided above. Instruments or devices necessary for administering the pharmaceutical composition(s) also may be included in the kits. For instance, a kit may provide one or more pre-filled syringes containing an amount of the anti-C5 antibody, or antigen-binding fragment thereof.

In one embodiment, the present invention provides a kit for reducing the likelihood of forming an allograft vasculopathy lesion in a recipient, the kit comprising: (a) a dose of an anti-C5 antibody, or antigen-binding fragment thereof, comprising CDR1, CDR2 and CDR3 domains of the heavy chain variable region having the sequence set forth in SEQ ID NO:12, and CDR1, CDR2 and CDR3 domains of the light chain variable region having the sequence set forth in SEQ ID NO:8; and (b) instructions for using the anti-C5 antibody or antigen-binding fragment thereof, according to any of the methods described herein. Optionally, the dose is contained in a pre-filled syringes.

### EXAMPLES

### Example 1

Longevity of cardiac (Beygui F et al. J Heart Lung Transplant 2010; 29: 316-322; Lee MS et al. Cardiac allograft vasculopathy Rev Cardiovasc Med 2011; 12: 143-152; Costello JP et al. Tex Heart Inst J 2013; 40: 395-399; Hollis IB et al. Pharmacotherapy 2015; 35: 489-501), renal (Bagnasco SM, Kraus ES. Curr Opin Organ Transplant 2015; 20: 343-347; Gupta G, et al. Transplantation 2014; 97: 1240-1246) and composite allografts (Mundinger GS, Drachenberg CB. Curr Opin Organ Transplant 2014; 19: 309-14; Unadkat JV et al. Am J Transplant 2009; 10: 251-261; Unadkat JV et al. Transplant Proc 2009; 41: 542-545) is frequently limited by allograft vasculopathy (AV), a condition characterized by irreversible stenoses and thromboses that develop throughout the graft vasculature. In the most well-appreciated form of AV, affected vessels contain a diffusely expanded neointima made up of smooth muscle-like cells along with sub-endothelial infiltrates of T cells and macrophages. Similar appearing neointimal lesions can be induced in human artery segments in response to human IFN-γ (Tellides G et al. Nature. 2000; 403: 207-11) or IFN-γ-producing human T cells. (Shiao SL et al, J Immunol 2007; 179: 4397-404). A second, more recently recognized form of AV involves the formation of thromboses, especially in the allograft microvasculature.

Donor specific antibody (DSA) as well as delayed graft function [a manifestation of ischemia reperfusion injury (IRI)] are risk factors for AV. We propose that these factors mediate AV by increasing the capacity of EC to stimulate IFN-γ production by alloreactive T cells. Using high titer panel reactive antibody (PRA) sera pooled from allosensitized transplant candidates we developed a protocol to bind alloantibody and activate complement, resulting in increased EC immunogenicity mediated in response to deposition of membrane attack complex (MAC) and non-canonical NF-κB signaling. In vivo, treatment of human artery segments with PRA led to human IgG binding to, murine MAC deposition on, and activation of, noncanonical NF-κB signaling in intimal EC. Jane-Wit D et al. Circulation. 2013; 128: 2504-2516. These effects resulted in exacerbated formation of T cell-mediated AV-like lesions. We have also shown that IRI-like lesions also exacerbate T cell-mediated AV in the same humanized mouse model.¹⁴ Our more recent studies have shown that inhibition of endocytosis blocked the effect of PRA treatment (Yi T, et al. Arterioscler Thromb Vase Biol 2012; 32: 353-360), but this is unlikely to be tolerated clinically; prevention of complement activation, an inducible process linked to inflammation appeared a more attractive alternative. Although complement activation has been linked to IRI (Miwa T et al. J Immunol 2013; 190: 3552-3559; Elvington A, et al. J Immunol 2012; 189: 4640-4647; Atkinson C et al. J Immunol 2010; 185: 7007-7013), we had not determined if terminal complement activation occurs in our humanized model of IRI. Eculizumab is a monoclonal anti-human C5 antibody that blocks the generation of downstream inflammatory mediators including C5a, a fluid-phase anaphylatoxin, and C5b, a terminal complement component which, along with C6, C7, C8, and polymers of C9, assemble to form solid-phase MAC. In this report, we investigated the effects of an anti-mouse C5 mAb, whose actions may be comparable to that of eculizumab, on non-canonical NF-κB signal activation in EC and the development of AV lesions using our humanized mouse models of alloantibody- and IRI-mediated AV.

### MATERIALS AND METHODS

### Examination of Effects of PRA on Human Vessel Grafts

All human materials were obtained under protocols approved by the Yale Human Investigations Committee or the IRB of the New England Organ Bank. All animal experiments were conducted under protocols approved by the Yale institutional Animal Care and Use Committee.

Discarded high-titer panel reactive antibody (PRA) sera were obtained from cardiac and renal transplant candidates as de-identified samples from Yale-New Haven Hospital's tissue typing laboratory. Sera from patients that had undergone panel reactive antibody (PRA) blood testing and found to have >80% reactivity to either HLA class I and/or class II antigens were pooled, tested and found to be negative for endotoxin activity (Sigma) prior to use. Human peripheral blood mononuclear cells (PBMC) were collected from healthy adult volunteer donors.

For PRA-mediated AV, adjacent 3-5 mm lengths of third or fourth order human coronary artery segments, approximating the caliber of murine aortae, were surgically implanted as end-to-end interposition grafts in the infrarenal position of descending aortae of paired SCID/bg immunodeficient mice (Taconic). The transplanted vessels were quiesced for ~30 days prior to i.v. tail injection of 200µL neat PRA sera (in one mouse) or PRA sera depleted of IgG using a mAb Trap serum fractionation kit (GE HealthCare) into the paired mouse. Eighteen hours later, grafts were harvested and immunostained. In other experiments, each of the paired human arterial xenografts was explanted with cuffs of mouse aorta on both ends and then interpositioned into the infrarenal aortae one member of a second pair of naive SCID/bg hosts that had been inoculated with human peripheral blood mononuclear cells.100-200×10⁶ cells allogeneic to the artery donor. In these mice, the efficiency of T cell engraftment was assessed by flow cytometry of peripheral blood sampled at weekly intervals, and the percentage of CD3⁺ engraftment relative to total murine CD45⁺ cells ranged between 5-15% prior to arterial xenograft implantation. Re-implanted grafts were harvested 14 days after implantation. Harvested tissues were frozen in OCT media blocks, sectioned at 5µm thickness, and subjected to morphologic, immunohistochemical, and immunofluorescent analyses as previously described. Jane-Wit D et al. Circulation. 2013; 128: 2504-2516.

**Induction of Ischemia-Reperfusion Injury (IRI).** As above, adjacent 3-5mm lengths of third or fourth order human coronary artery segments approximating the caliber of murine aortae were surgically implanted as end-to-end interposition grafts in the infrarenal position of descending aortae of SCID/bg immunodeficient mice (Taconic). Following a ~1 month period of quiescence, arterial xenografts along with cuffs of mouse aorta on both ends were explanted and incubated ex *vivo* under conditions of anoxia for 12 h prior to surgical reimplantation into a second pair of SCID/bg recipients and analyze 18 h later. Alternatively, where indicated, arterial xenografts subjected to anoxia were reimplanted into SCID/bg hosts that had been inoculated with 100-200x106 human peripheral blood mononuclear cells and grafts were harvested 21 days after implantation or sooner if evidence was seen of distress or hindlimb paralysis indicative of thrombosis.

**Anti-C5 and Anti-C5a Blocking Antibodies.** Anti-mouse C5 blocking antibody (BB5.1), control isotype antibody (12B4), anti-human C5a blocking antibody (CLS026) and control murine isotype antibody (MOPC1) were provided by Alexion Pharmaceuticals. Mice were injected subcutaneously with 0.8 mg of each antibody or 0.8mg of each antibody were added to hypoxic media prior to surgical implantation as specified in the text and figure legends.

**Statistical Analyses.** All experiments involved comparisons between pairs of animals receiving human artery segments from adjacent portions of the same donor vessel subjected to distinct manipulations. Statistical analyses were performed using computer software (Origin), and the data were analyzed by two-tailed Student t tests where P<0.05 was considered significant.

**Flow Cytometry.** Human umbilical vein endothelial cells (HUVEC) were cultured in 24 well microtiter plates precoated with 0.1% gelatin. HUVEC were treated with IFN-y (50ng/mL) in EGM2 media (Lonza) for 72 h prior to washing with warm gelatin veronal buffer (Sigma) and addition of BB5.1 or 12B4 mAbs (25µg). Thirty minutes later, the IgG+ fraction of PRA sera was added at 1:4 dilution v/v along with either 1:10 dilution v/v mouse complement (Sigma) or human complement (Sigma) for 4 h prior to FACS analysis. The final volume in all wells was 200µL. Cells were analyzed using a FACS Caliber flow cytometer (Becton Dickinson).

**Serum Fractionation for in vitro activation of complement.** Fractionation of PRA sera into IgG+ and IgG⁻ components were performed as previously described. Jane-Wit D, et al. Circulation. 2013; 128: 2504-2516. Briefly, total IgG concentrations were first determined in intact sera prior to fractionation by ELISA (Invitrogen). Then, per manufacturer's specifications using a MAbTrap Kit (GE Healthcare, Piscataway, NJ), 500µL of neat sera were diluted 1:1 in binding buffer and passed through the provided column pre-equilibrated with binding buffer. The IgG⁻ fraction was then collected. Subsequently, the column was washed, and the IgG+ fraction was eluted from the column. Buffer exchange from elution buffer to sterile phosphate buffered saline (Gibco) was performed by multiple centrifugations x5 using Amicon Ultra Centrifugal Filter Devices (EMD Millipore) with a 20kDal cutoff. All IgG+ fractions were brought to a final concentration equivalent to the total IgG concentration prior to sera fractionation. All isolated fractions were then used at 1:4 dilution v/v in gelatin veronal buffer.

**Plasma C5a Quantification.** Mouse plasma samples were collected in heparinized tubes at the time of graft harvest from PRA-treated animals bearing human artery grafts, and C5a bound to circulating immune complexes was separated by incubation with protein A agarose. Mouse heparinized plasma was added to a solution containing 1% Blocker A (Meso-Scale Discovery, Cat #R93AA-1) containing 20mM disodium EDTA and 100µg/mL FUT-175, a broad-spectrum complement inhibitor (BD Biosciences, Cat #552035) in PBS pH 7.4 in 1.5 mL microcentrifuge tubes. Protein A Plus Agarose beads (Thermo Fisher Scientific, Cat #22812) was added to achieve a ~50% slurry. The mixture was centrifuged at 4°C at 7000g for 10 seconds. The supernatant (~50µL) was removed and 120µL of PBS pH 7.4 solution containing 1% blocker A, 20mM EDTA, and 100µg/mL FUT-175 was added along with 30µL of mouse heparin. The mixture was slowly rotated for 1 hour at 4°C. The above steps involving incubation with Protein A Plus Agarose beads was repeated a second time prior to centrifugation at 4°C to ensure complete immune complex removal prior to use in the C5a immunoassay below.

Following immune complex removal, mouse plasma C5a levels were assessed by immunoassay. For this assay Meso-Scale Discovery (MSD) plate (Cat #L15XB) was coated with anti-mouse C5a mAb (clone p5aN195) at 2µg/mL in PBS at 4°C overnight. Plates were washed with PBS, blocked with 3% MSD Blocker A for 30 minutes at room temperature, and washed with PBS containing 0.05% Tween-20, Standards were made in triplicate by adding 25µL C5a (R&D Systems, Cat #07-202-503) to 75µL C5 deficient mouse plasma in 1% MSD Blocker A containing 20mM EDTA and 100µg/mL FUT-175 (BD Biosciences, Cat #552035), and then serially diluting from 10,000pg/mL down to 2.4pg/mL. Samples were then diluted 1:100 in 1% MSD Blocker A containing 20mM EDTA and 100µg/mL FUT-175 and added at 25µL/well for 1 hour at room temperature. Plates were then washed with PBS, and 25µL of biotinylated rat anti-mouse C5a (Cat #558028) at 2µg/mL plus strepavidin-SULFO-TAG (Meso-Scale Discovery, Cat #R32AD-5) at 1µg/mL was added in 1% Blocker A for 1 hour at room temperature with gentle shaking. Plates were washed, and 1X MSD Read Buffer T (Cat #R92TC-2) was added at 150µL/well. Plates were read using a Meso-Scale Discovery Sector 600 Imager and analyzed using Meso-Scale Discovery Workbench 4.0 software.

### Humanized Model of Alloantibody-Induced AV

For testing effects of PRA in vivo, adjacent 3-5 mm lengths of third or fourth order human coronary artery segments, approximating the caliber of murine aortae, were surgically implanted as end-to-end interposition grafts in the infrarenal position of descending aortae of paired 8-12 week old female C.B-17 SCID/bg immunodeficient mice (Taconic). The transplanted vessels were quiesced for ~30 days prior to i.v. tail injection of 200 µL neat PRA sera. In experiments involving short term effects, grafts were harvested 18 h later and immunostained. For experiments to assess effects of PRA on development of AV, each of the paired human arterial xenografts was explanted with cuffs of mouse aorta on both ends and then interpositioned into the infrarenal aortae one member of a second pair of naive SCID/bg hosts that had been inoculated with 100-200×10⁶ human PBMC allogeneic to the artery donor. In these mice, the efficiency of T cell engraftment was assessed by flow cytometry of peripheral blood sampled at weekly intervals, and the percentage of CD3⁺ engraftment relative to total murine CD45⁺ cells ranged between 5-15% prior to arterial xenograft implantation. Re-implanted grafts were harvested 14 days after implantation.

**Analysis of Graft Vessels.** Harvested human artery grafts were flash frozen in OCT media blocks, sectioned at 5µm thickness, and fixed and permeabilized with ice cold methanol for 15 minutes at 4°C. Sections were blocked with PBS containing 0.1% Tween-20 and 5% fetal bovine serum for 2 hours at room temperature prior to staining at 4°C overnight using 1:100 dilution of the following antibodies: biotinylated Ulex Europeus Agglutinin I (Vector Labs, Burlingame, VT), anti-human CD31 (Dako), anti-smooth muscle actin (Sigma, clone 1A4), anti-human CD45RO (eBioscience, clone UCHL1), anti-mouse anti-Gr-1 (BD Biosciences, catalog #550291), anti-C5 Ab (abcam), anti-C5b-9 (Dako), anti-human VCAM-1 (Novus, clone 6G9). Polyclonal rabbit anti-mouse C4d (gift of Dr. William Baldwin III, Cleveland Clinic) was used at 1:50 dilution and detected using strepavidin-conjugated Alexa Fluor 488, goat anti-mouse Alexa Fluor 488, donkey anti-mouse Alex Fluor 546, or donkey anti-rabbit Alex Fluor 594 secondary antibodies at 1:200 dilution (Invitrogen) at room temperature for one hour. Hypoxyprobe was used at 1:200 dilution according to the manufacturer's specifications. Neointimal areas, lumen size, and medial thickness were calculated as previously described. Yi T, et al. Arterioscler Thromb Vase Biol 2012; 32: 353-360.

### RESULTS

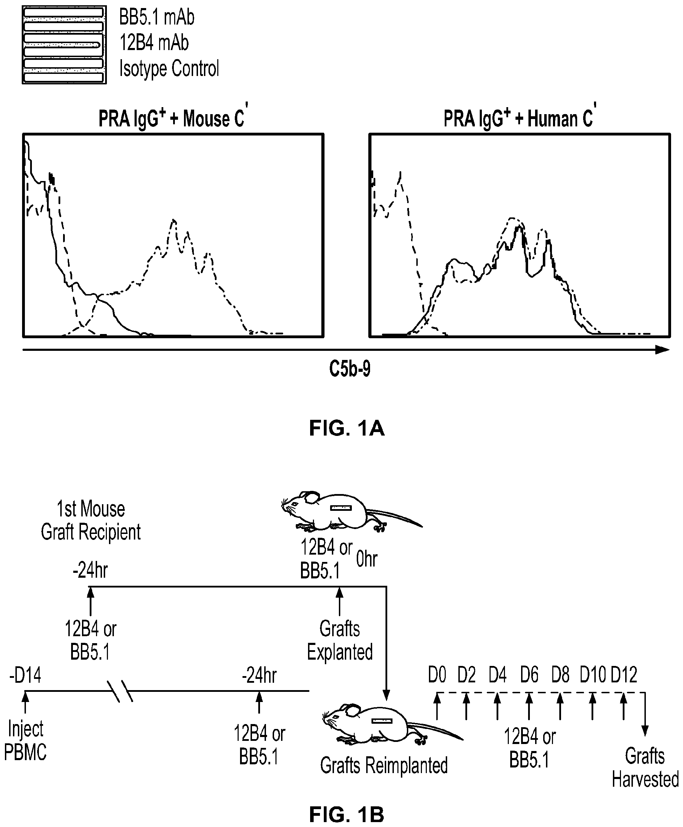

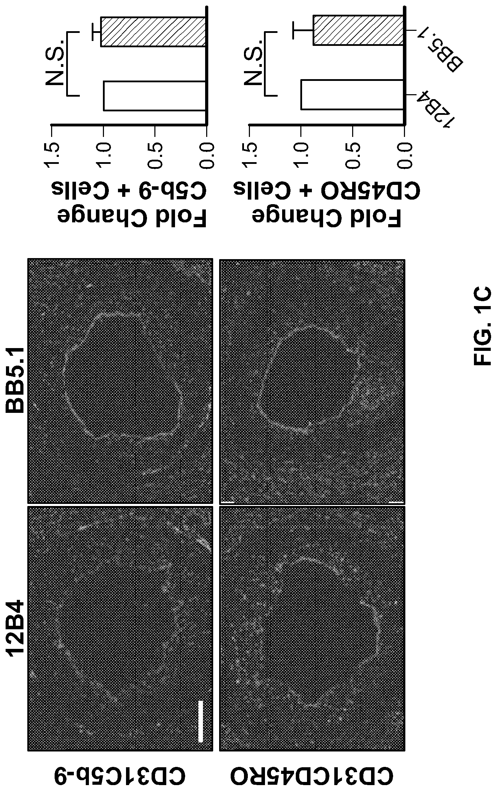

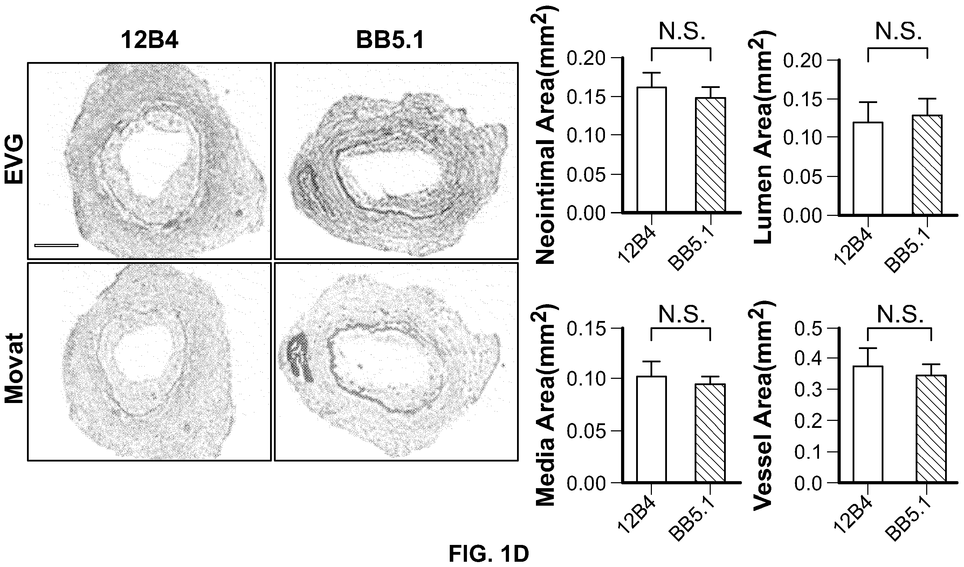

**FIG. 1** shows the results of an experiment demonstrating that anti-C5 mAb does not exert immunomodulatory effects on T cell-mediated AV in the absence of terminal complement activation. HUVEC were pretreated with 12B4 control mAb or BB5.1 anti-C5 mAb (25µg) for 30 minutes prior to the addition of the IgG+ fraction of PRA and mouse complement (C') or human complement for 4h. Cells were then analyzed by FACS for C5b-9 (a). Quiesced human coronary artery grafts in immunodeficient mice were pre-exposed to 0.8mg 12B4 or BB5.1 antibody, and at the time of graft harvest 12B4 or BB5.1 mAbs were added into a PBS media bath and immediately retransplanted into a second host which had been pre-treated with 12B4 or BB5.1 mAbs and contained circulating human T cells from a prior adoptive transfer of PBMC allogeneic to the artery donor. The second hosts received ongoing 12B4 or BB5.1 Ab treatments at 0.8mg/injection/mouse for 14 days as shown (b). Arterial grafts were analyzed for C5b-9 (c, top row) staining and CD45RO+ T cell infiltration (c, bottom row). Neointimal lesion formation was assessed between treatment groups following EVG and Movat staining (d). n=5 treatment pairs for all experiments. No significant differences between control and specific anti-C5 antibody were seen in the absence of terminal complement activating stimuli (N.S.). Scale bars indicate 63µm.

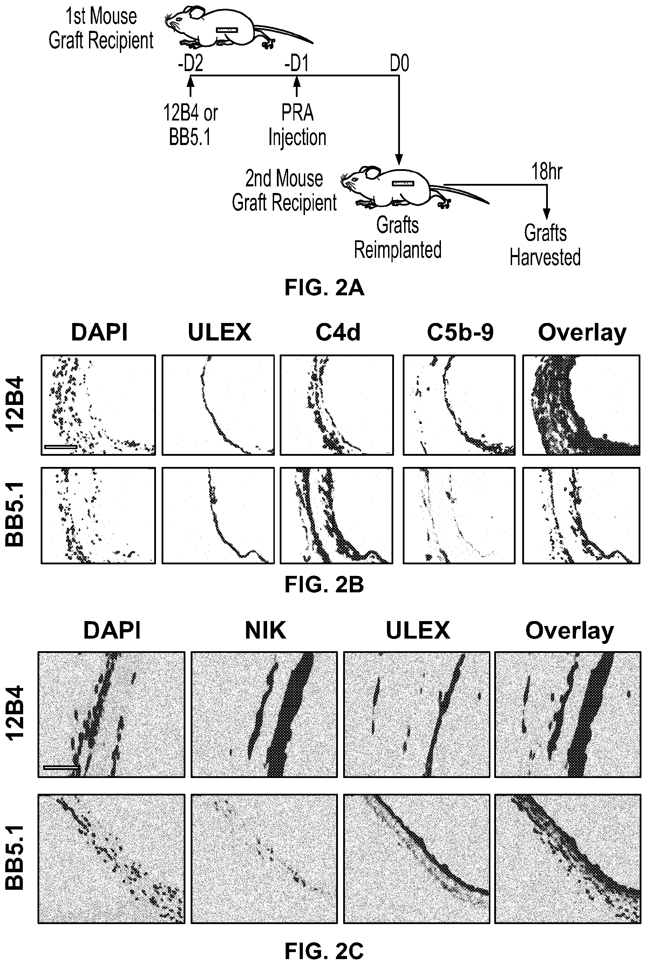

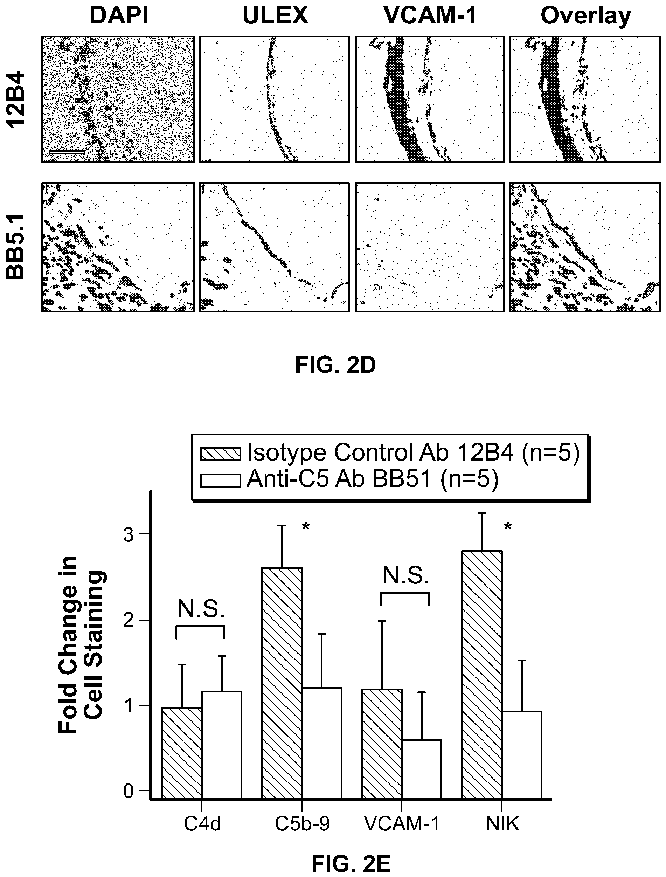

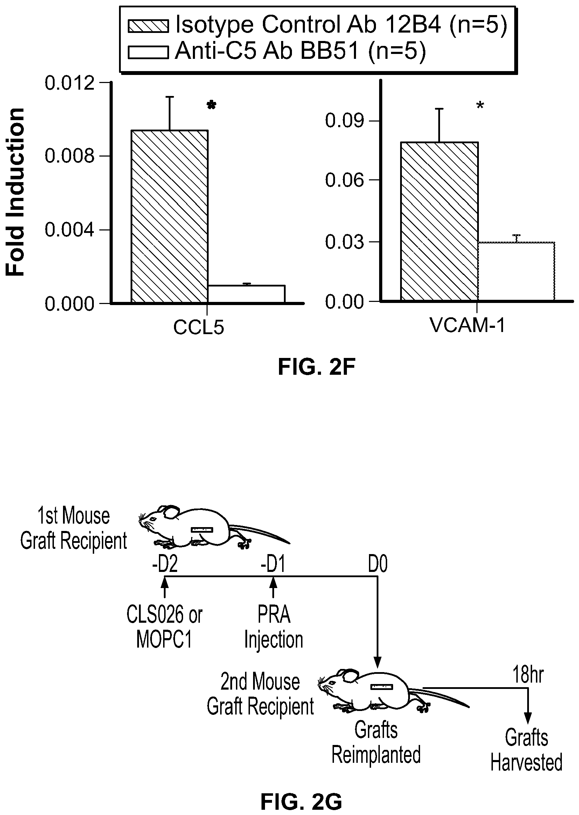

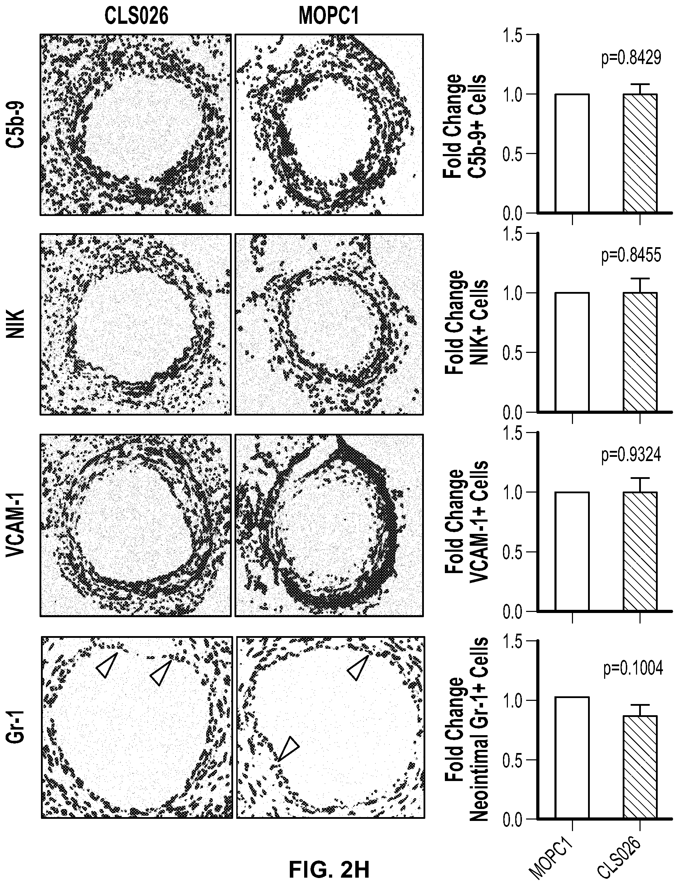

**FIG. 2** shows the results of an experiment demonstrating that the blockade of MAC assembly and not C5a generation by Anti-C5 mAb attenuates EC activation in response to DSA. To assess the effect of anti-C5 treatment on alloantibody-induced complement activation, SCID/bg immunodeficient hosts were treated with isotype control 12B4 mAb or anti-C5 BB5.1 mAb prior to i.v. tail vein injection of PRA, and grafts were harvested 18 hours later (a). Compared to 12B4 Ab-treated hosts, animals treated with BB5.1 Ab showed equivalent C4d staining but decreased C5b-9 staining (b). BB5.1 mAb treatment of coronary xenografts showed decreased intimal NIK staining (c) and VCAM-1 expression (d) compared to 12B4 Ab-treated controls. C4d, C5b-9, NIK, and VCAM-1 staining results were quantified (e). Intra-graft transcripts of CCL5 and VCAM-1 were quantified and normalized to CD31 transcript levels (f). n=5 treatment pairs for the above experiments. SCID/bg hosts were treated as shown with isotype control MOPC1 or anti-C5a CLS026 mAb prior to i.v. tail vein injection of PRA and graft harvest 18 hours later (g). Compared to hosts treated with control MOPC1 mAb, CLS026 mAb-treated hosts developed equivalent levels of C5b-9 deposition (h, top row), NIK upregulation (h, second row), VCAM-1 expression (h, third row), and neointimal recruitment of Gr-1⁺ cells (h, arrowheads, bottom row). n=3 treatment pairs for the above experiments. N.S. indicates no statistical difference between groups. Asterisks indicate p<0.05. Scale bars indicate 135µm.

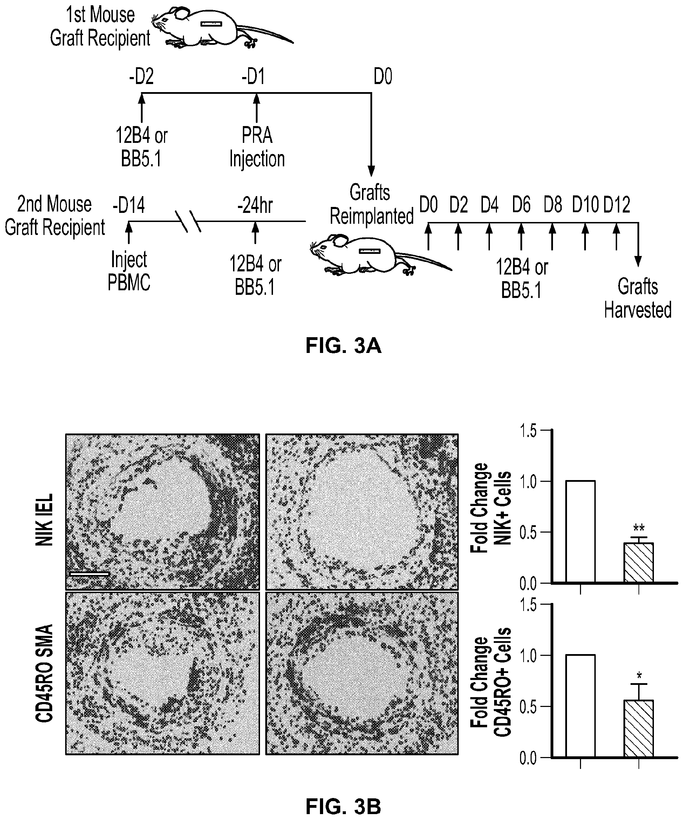

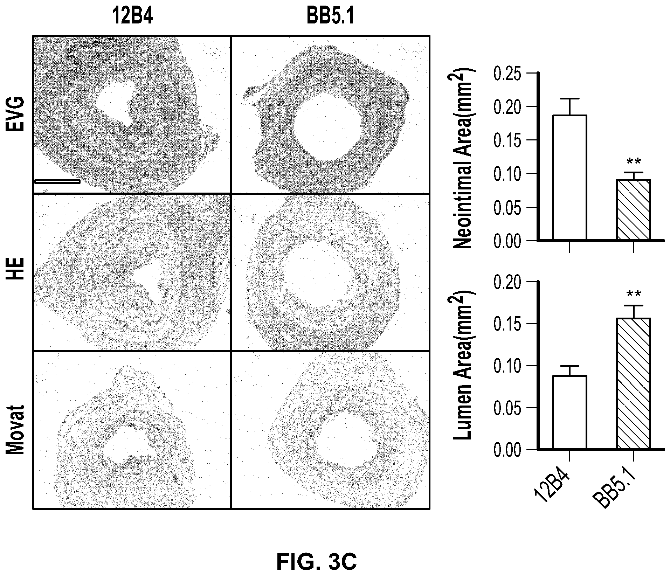

**FIG. 3** shows the results of an experiment demonstrating that anti-C5 antibody reduces neointimal lesions following alloantibody-induced complement activation. 12B4 isotype control or BB5.1 anti-C5 Ab were tested for their effects on alloantibody-induced complement activation and neointimal formation. Arterial grafts were pre-treated with 12B4 or BB5.1 Ab prior to i.v. PRA injection and reimplantation into a second immunodeficient hosts engrafted with human T cells and similarly pre-treated with 12B4 or BB5.1 Ab. These hosts additionally received ongoing antibody treatments for 14 days prior to graft harvesting (a). Compared to 12B4 Ab-treated controls, hosts treated with BB5.1 mAb showed attenuated intimal NIK staining (b, top row) and intimal infiltration of CD45RO+ alloimmune T cells (b, bottom row). Neointimal and luminal areas were quantified in 12B4⁻ and BB5.1 mAb-treated hosts (c). n=6 treatment pairs for all experiments. IEL indicates internal elastic lamina. Asterisks indicate p<0.05. Scale bars indicate 63µm.

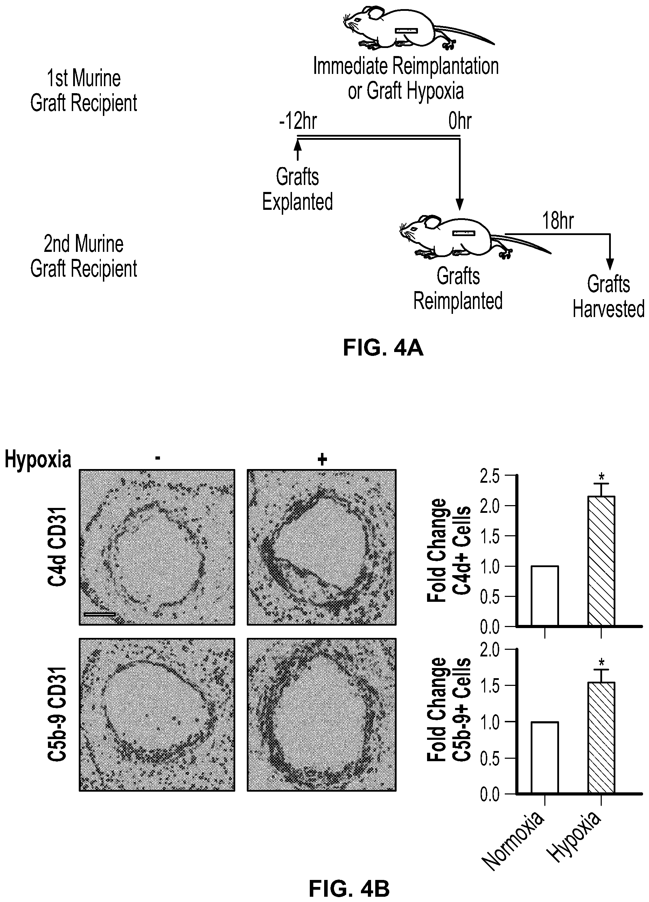

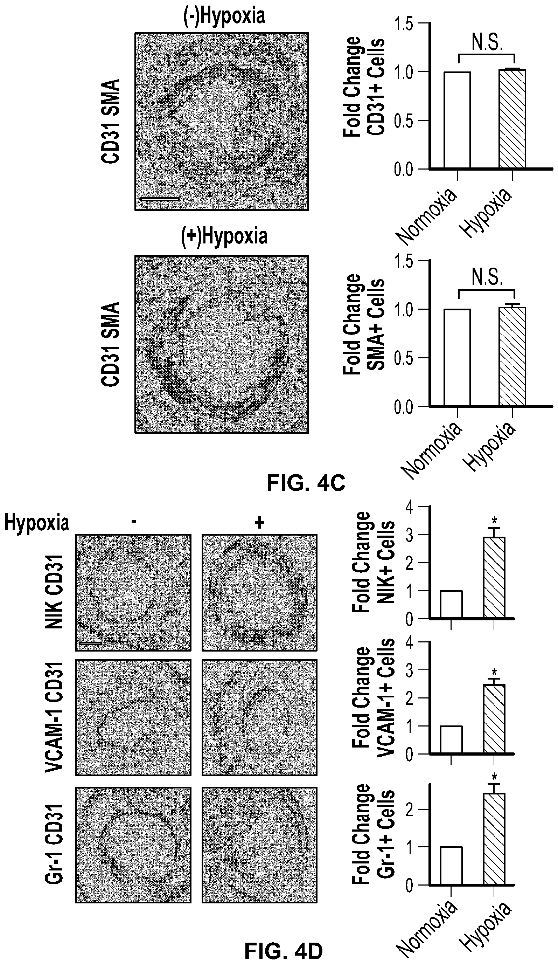

**FIG. 4** shows the results of an experiment demonstrating that IRI activates complement and induces MAC formation in the artery wall without overt target cell loss. For IRI-induced complement activation, coronary artery xenografts parked in an immunodeficient host were explanted and either immediately surgically reimplanted into a second immunodeficient host [(-) hypoxia] or placed in organ culture under hypoxic conditions for 12 hours [(+) hypoxia] prior to reimplantation (a). Explanted coronary arteries subjected to 12 hours of hypoxia ex vivo were examined by I.F. for CD31 and either C4d (b, top row) or C5b-9 (b, bottom row) and staining results were quantified as indicated. CD31 and SMA staining were quantified between control grafts not subjected to hypoxia or grafts subjected to 12 hours of hypoxia (c). NIK (d, top row), VCAM-1 (d, middle row), and Gr-1+ (d, bottom row) staining were performed in coronary grafts and results were quantified as indicated. n=6 treatment pairs for all experiments. N.S. indicates no statistical difference between groups. Asterisks indicate p<0.05. Double asterisks indicate p<0.001. Scale bars indicate 63µm.

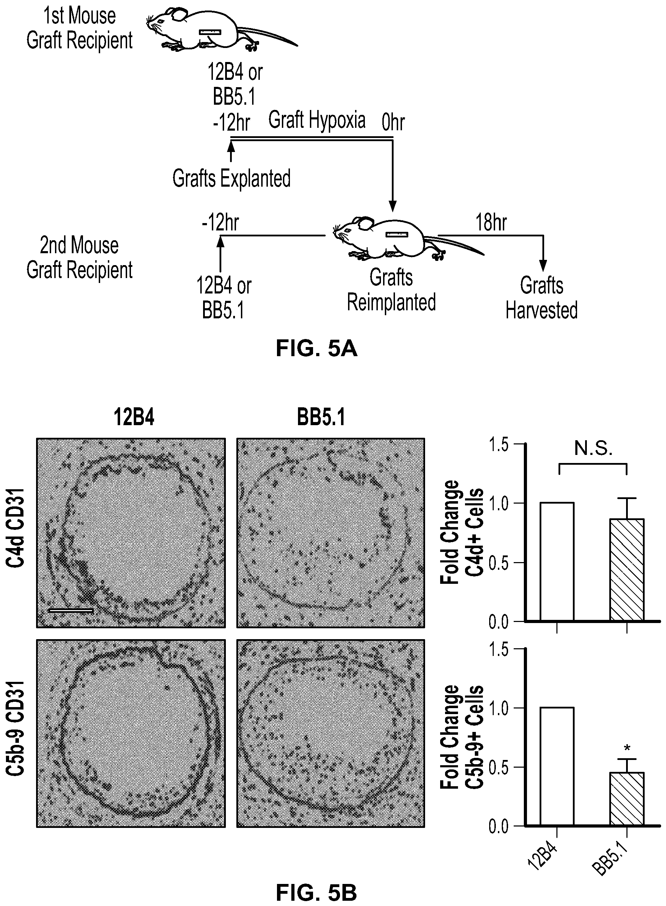

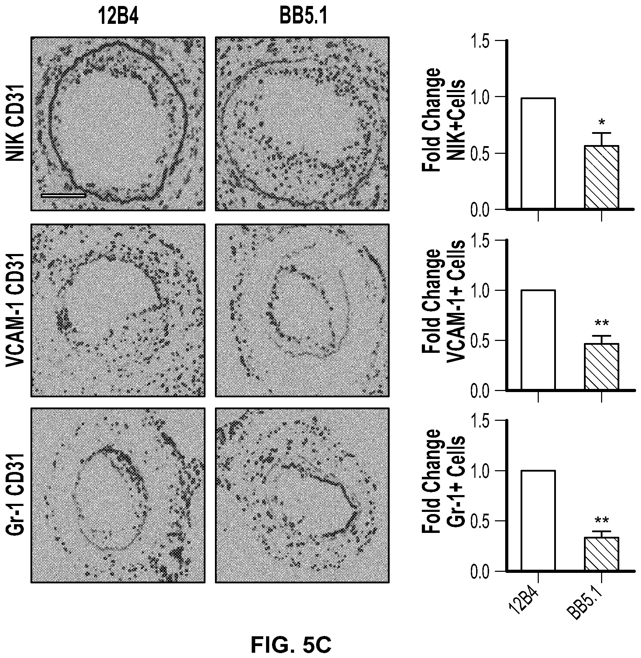

**FIG. 5** shows the results of an experiment demonstrating that terminal complement inhibition blocks IRI-induced MAC formation, non-canonical NF-κB, and EC activation. Coronary artery segments were exposed to 12B4 or BB5.1 mAb and subjected to ex vivo hypoxia as shown prior to graft harvesting 18 hours later (a). Explanted coronary artery xenografts were analyzed for early and terminal complement activation, with C4d (b, top row) and C5b-9 (b, bottom row), respectively. Grafts were additionally analyzed for EC activation with NIK (c, top row), VCAM-1 (c, middle row), and Gr-1 (c, bottom row). n=4 treatment pairs for all experiments. N.S. indicates no statistical difference between groups. Asterisks indicate p<0.05. Double asterisks indicate p<0.001. Scale bars indicate 63µm.

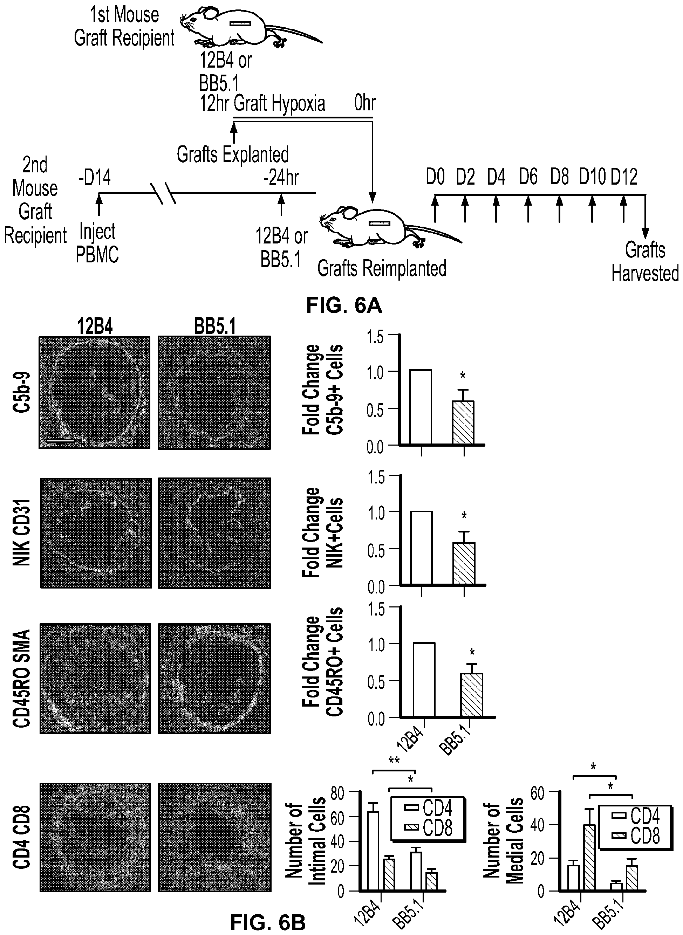

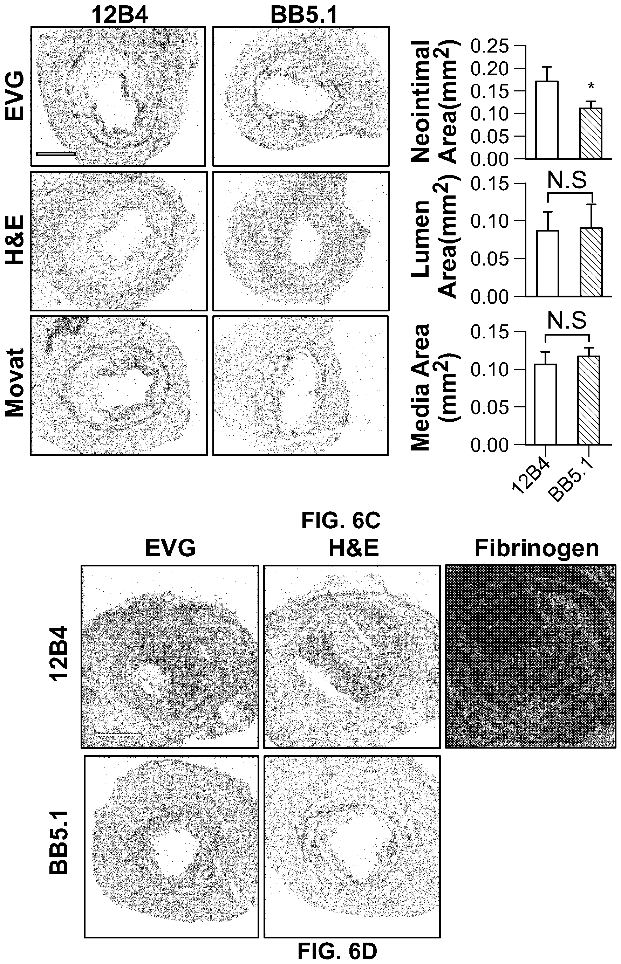

**FIG. 6** shows the results of an experiment demonstrating that anti-C5 antibody attenuates neointimal and thrombotic AV lesions following IRI. Anti-C5 therapy was tested for its effects on IRI-induced complement activation and development of AV lesions. Human arterial xenografts were pretreated with either 12B4 or BB5.1 Ab prior to graft explanation, exposure to hypoxia for 12 hours, and reimplantation into a second immunodeficient host that had been pre-treated with 12B4 or BB5.1 mAb and engrafted with human T cells. These hosts received mAb treatment as shown (a). Effects of anti-C5 Ab were assessed in coronary artery xenografts exposed to IR injury. Compared to 12B4 Ab-treated controls, hosts given BB5.1 Ab showed significantly decreased intimal and medial staining of C5b-9 (b, top row), NIK staining (b, second row), and a significant reduction in the number of CD45RO+ infiltrating T cells (b, third row). Intimal- and medial-infiltrating CD4+ and CD8+ T cells were quantified following 12B4 or BB5.1 mAb treatment (b, bottom row). Neointimal thickness, luminal area, and medial thickness were quantified in hosts receiving 12B4 or BB5.1 treatments (c). Intraluminal thrombosis was visualized in 3 of 8 hosts treated with 12B4 Ab (d, top row) and 0 of 8 hosts treated with BB5.1 Ab (d, bottom row). n=8 treatment pairs for all experiments. N.S. indicates no statistical difference between groups. Asterisks indicate p<0.05. Double asterisks indicate p<0.001. Scale bars indicate 63µm.

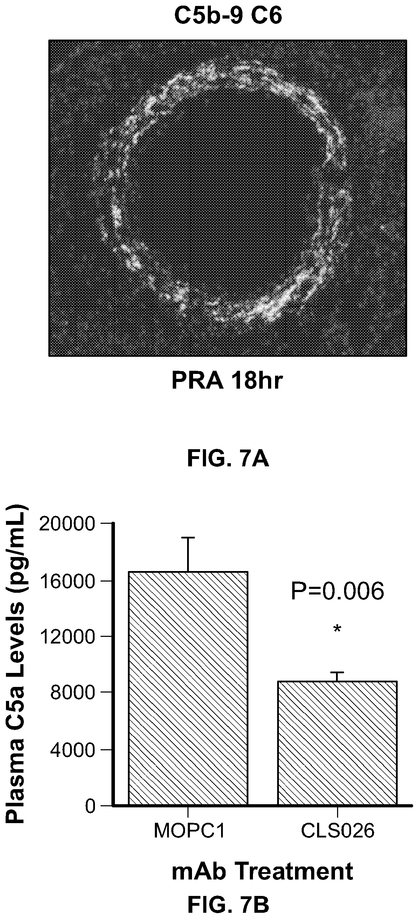

**FIG. 7** shows the results for naive SCID/bg mice containing coronary artery interposition grafts implanted in the descending aorta received i.v. injection of PRA sera prior to graft harvest 18 hours later and analysis by I.F. (n=3 treatment pairs). C5a activity levels were assessed in murine plasma at the time of graft harvest as depicted in Fig 2g following treatment with blocking anti-C5a mAb CLS026 or an isotype control mAb MOPC1 (n=3 treatment pairs).

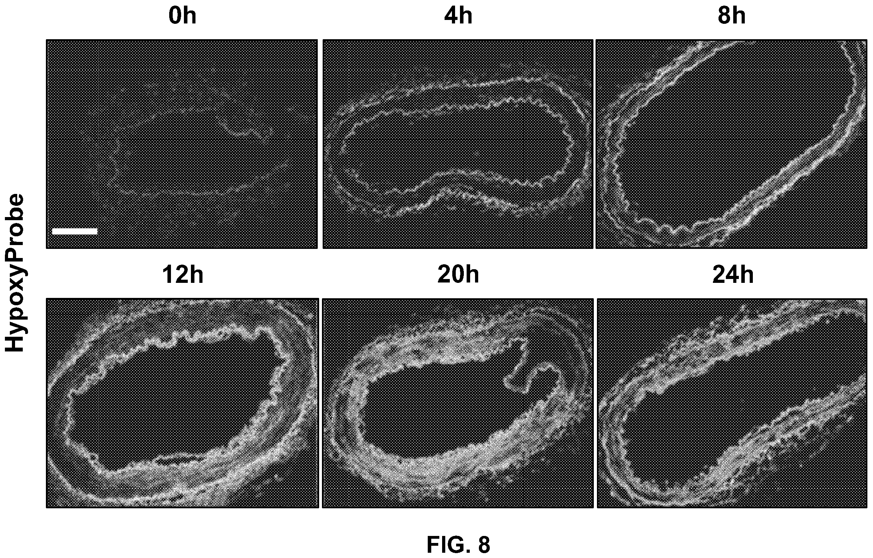

**FIG. 8** shows coronary artery grafts were explanted, placed in organ culture under anoxic conditions for the times indicated and stained for hypoxyprobe (n=5 mice for each time point). Scale bars indicate 63µm.

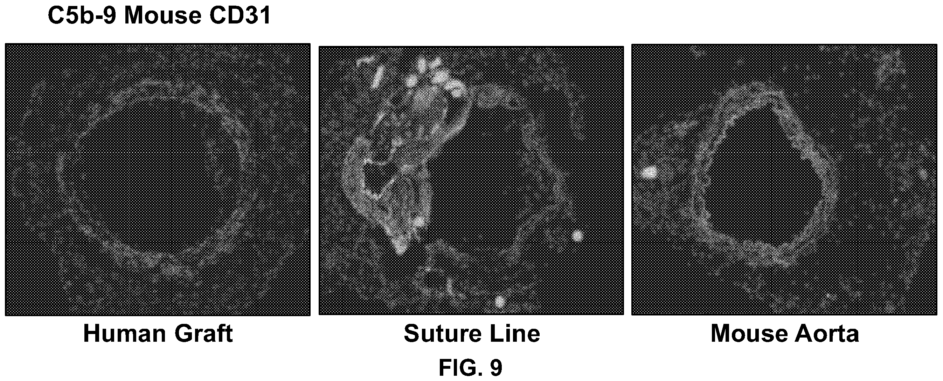

**FIG. 9** shows coronary artery grafts were explanted, placed in organ culture under anoxic conditions for 12 hours and then reimplanted into a second set of naive SCID/bg hosts. Grafts were harvested 18 hours after re-implantation and serial sections were taken for I.F. analysis adjacent to the distal suture line, indicating the margin between human arterial graft and murine descending aorta tissue.

**Anti-C5 mAb Does Not Exert Immunomodulatory Effects on T Cell-Mediated AV in the Absence of Terminal Complement Activation.** Prior to use in vivo, the efficacy of BB5.1 mAb for inhibiting terminal complement activation and its purported specificity for selectively inhibiting murine complement was confirmed in vitro. To do this we exploited the fact that the IgG⁺ fraction of panel reactive antibody (PRA) sera from allosensitized transplant candidates caused IgG binding to EC, a process that could elicit terminal activation of murine or human complement. Jane-Wit D et al. Circulation. 2013; 128: 2504-2516. We thus treated human umbilical vein EC (HUVEC) with the IgG⁺ fraction of PRA sera in the presence of exogenous human or murine complement and assessed for surface deposition of C5b-9 by flow cytometry. We found that, compared to 12B4 mAb-treated EC, BB5.1 mAb blocked the ability of the IgG⁺ fraction of PRA sera to elicit C5b-9 deposition in the presence of murine complement (**FIG. 1a**, Left) but not human complement (**FIG. 1a****,** right). In contrast, C4d staining was unchanged by 12B4 or BB5.1 mAb (data not shown). These data show that BB5.1 mAb could effectively and selectively inhibit terminal activation of murine complement.

We next assessed the effects of anti-mouse C5 mAb BB5.1 on T cell-mediated formation of AV lesions in our humanized mouse model in the absence of exacerbating factors, i.e., DSA or IRI, causing complement activation. Murine hosts engrafted with human T cells received control 12B4 or anti-C5 BB5.1 mAbs as diagrammed in **FIG. 1b****.** We did not observe complement activation in this protocol in either treatment group as indicated by a lack of staining of C5b-9, the major component of MAC **(****FIG. 1c****,** top row). Additionally, there were no significant differences in CD45RO+ T cell infiltration **(****FIG. 1c****,** bottom row) or neointima lesion area (**FIG. 1d**) between hosts treated with anti-C5 mAb or isotype control mAb.

Together these data indicate that anti-C5 BB5.1 mAb does not non-specifically attenuate graft inflammation or neointimal lesion formation in the absence of complement activation.

**Blockade of MAC Assembly and Not C5a Generation by Anti-C5 mAb Attenuates EC Activation in Response to Alloantibody.** Exposure of human arterial grafts to PRA *in vivo* leads to human IgG binding to EC, thus modeling the effect of DSA. IgG binding to EC is followed by murine complement activation leading to MAC assembly on the EC13 in SCID/bg hosts; this strain has complement activity comparable to immunocompetent mouse strains. Cragg MS, Glennie MJ. Blood. 2004; 103: 2738-2743. PRA treatment induced non-canonical NF-κB signaling as detected by NIK stabilization, a MAC-dependent effector pathway causing EC activation. Jane-Wit D et al. Circulation. 2013; 128: 2504-2516. We thus hypothesized that inhibition of terminal complement activation by anti-C5 mAb, sparing early complement activation, would subvert the proinflammatory changes in EC mediated through non-canonical NF-κB signaling. To test this, hosts bearing arterial xenografts were pre-treated with 12B4 or BB5.1 Ab prior to i.v. PRA injection. Following injection, grafts were harvested 18 hours later **(****FIG. 2a****).** Animals treated with BB5.1 mAb showed early (C4d) but not terminal complement activation, i.e., MAC assembly, as indicated by C9 staining which was confined to the intimal lining **(****FIG. 2b****).** To confirm MAC assembly, we co-stained PRA-treated grafts with C6, which, expectedly showed a high degree of neointimal co-localization with C9 **(****FIG. 7a****).** Hereafter, we will refer to the detected complexes as C5b-9. In contrast to BB5.1-treated animals, both C4d and C5b-9 deposition were detected in 12B4 control mAb-treated animals. Decreased C5b-9 staining in BB5.1-mAb-treated hosts correlated with significantly attenuated EC expression of NIK **(****FIG. 2c****,** **FIG. 2e****)** and VCAM-1 **(****FIG. 2d, FIG. 2e****),** markers of non-canonical NF-κB activation and inflammatory gene expression, respectively. Real-time PCR of graft lysates showed a significant reduction in NIK-dependent inflammatory genes, CCL5 and VCAM-1 **(****FIG. 2f****).**

These data demonstrate the efficacy of anti-C5 mAb in blocking terminal complement activation by PRA and its attendant inflammatory effects including non-canonical NF-κB signaling and EC activation.

BB5.1 prevents generation of both C5a and C5b. While our prior in vitro studies demonstrated that MAC (C5b-9), and not C5a, led to EC activation and increased immunogenicity, it is possible that C5a played some role *in vivo.* To determine which of these factors was the functionally relevant target of anti-C5 mAb, we treated hosts bearing human artery grafts with a mAb specifically blocking C5a (CLS026) or an isotype control mAb (MOPC1), as shown in **FIG. 2g****,** prior to injection with PRA. C5a activity was assessed in murine sera collected at the time of graft harvest. PRA treatment led to circulating mouse C5a and this was significantly inhibited in CLS026-treated hosts (Fig 7b) to levels observed in historical control mouse strains (data not shown). Importantly, inhibition of anti-C5a did not reduce the extent of intramural MAC assembly compared to controls **(****FIG. 2h****,** top row) and resulted in unchanged degrees of NIK upregulation, EC activation, and EC-mediated recruitment of Gr-1+ neutrophils in hosts treated with anti-C5a mAb vs control mAb **(****FIG. 2h****).**

As C5a blockade did not affect parameters of EC activation, we conclude from these data that MAC is primarily responsible for PRA-induced EC activation and, accordingly, the ability of anti-C5 mAb to attenuate EC activation is due to the ability of this mAb to block the assembly of MAC rather than the generation of C5a.

**Anti-C5 Antibody Reduces Neointimal Lesions Following DSA-Induced Complement Activation.** Having established the complement-blocking efficacy of anti-C5 mAb in PRA-treated animals, we then assessed the effect of anti-C5 mAb on alloantibody-mediated increases in neointimal AV lesions. Grafts pre-treated with either 12B4 or BB5.1 were exposed to PRA sera and then reimplanted into a second set of immunodeficient hosts that had been previously engrafted with human T cells and pre-treated with 12B4 or BB5.1 Ab. These second set of hosts then received ongoing antibody treatments for 14 days prior to graft harvesting as depicted in **FIG. 3a**. Compared to control hosts receiving 12B4 mAb, BB5.1 mAb-treated hosts showed significantly decreased intimal NIK staining **(****FIG. 3b****,** top row) and decreased infiltration of CD45RO+ alloimmune T cells **(****FIG. 3b****,** bottom row). These changes were associated with significantly decreased neointimal areas and increased luminal areas in BB5.1 mAb-treated hosts compared to 12B4 mAb-treated controls **(****FIG. 3c****).** No hosts in either the BB5.1- or 12B4-treated groups developed thrombotic AV lesions. These data showed that, in a complement-dependent model of alloantibody-induced AV, anti-C5 blocking antibody inhibited terminal complement activation, EC activation, and non-canonical NF-κB and that these changes correlated with significantly reduced neointimal AV lesion formation.

**IRI Activates Complement and Induces MAC Formation in the Artery Wall without Overt Target Cell Loss.** Activation of terminal complement has been shown in other models to contribute to IRI. Miwa T. et al., J Immunol 2013; 190: 3552-3559; Elvington A, Atkinson C et al., J Immunol 2012; 189: 4640-4647 ; Atkinson C, He S, Morris K, Qiao F, Casey S, Goddard M, et al. J Immunol 2010; 185: 7007-7013. We used an anti-C5 mAb therapy to test the contribution of terminal complement activation in our model of IRI14 where a role for terminal complement and non-canonical NF-κB had not been previously established. Before embarking on the current experiments, we re-evaluated the optimal time of hypoxia using human artery segments and found that 12 h led to more pronounced staining with hypoxyprobe **(****FIG. 8****)** without evidence of cell necrosis or aneurysm development upon re-transplantation. All subsequent experiments used 12 h of ex vivo hypoxia to trigger IRI. Using this adapted protocol as shown in **FIG. 4a**, we examined whether early (C4d) or terminal (C5b-9) complement activation occurred in arterial xenografts following 12 hours of ex *vivo* hypoxia relative to control grafts which were immediately reimplanted into second recipient hosts. In contrast to our model of PRA-induced coronary AV where complement staining was confined exclusively to EC **(****FIG. 2b****),** coronary artery xenografts exposed to hypoxia showed significantly increased C4d and C5b-9 deposition in both the intimal and medial regions of the vessel wall **(****FIG. 4b****),** consistent with prior observations that IRI affected mural smooth muscle cells as well as luminal EC.14 We then assessed whether IRI had caused non-specific complement activation on murine EC outside of human arterial xenografts by assessing C5b-9 staining in serial sections contiguous to the distal surgical suture line. In IRI-treated grafts we found that C5b-9 staining, while abundant on human intima and media proximal to the suture line, was completely absent in murine EC distal to the suture line, indicating that complement activation specifically occurred on human IRI-treated tissue (Fig S3). We furthermore did not observe loss of EC (CD31) or SMC (smooth muscle α-actin) in regions of MAC assembly **(****FIG. 4c****),** consistent with non-lethal injury. We also assessed downstream effects of terminal complement activation. We observed significantly increased NIK protein expression throughout the vessel wall, co-localizing with MAC and no longer restricted to EC, **(****FIG. 4d****,** top row). NIK expression correlated with significantly increased VCAM-1 **(****FIG. 4d****,** second row) and recruitment of mouse Gr1+ neutrophils **(****FIG. 4d****,** bottom row).

These data suggest that IRI activated non-canonical NF-κB signaling in both EC and SMC, a process correlating with EC activation and acute mural inflammation. Our prior study showed that in recipient animals lacking human T cells, this episode of perioperative acute inflammation resolves without long term sequelae. Yi T, et al. Arterioscler Thromb Vase Biol 2012; 32: 353-360.

**Terminal Complement Inhibition Blocks IRI-Induced MAC Formation, Non-Canonical NF-κB Signaling, and EC Activation.** We investigated the effects of BB5.1 mAb in our humanized model of IRI-exacerbated AV. We first examined whether BB5.1 could block terminal complement activation in this model as shown in Fig 5a. Compared to hosts treated with 12B4 control mAb, hosts treated with BB5.1 mAb showed early (C4d, **FIG. 5b**, top row) but not terminal (C5b-9, **FIG. 5b****,** bottom row) complement activation following induction of IRI. Blockade of terminal complement was associated with significantly decreased NIK (Fig 5c, top row), VCAM-1 expression **(****FIG. 5c****,** middle row), and decreased infiltration of mouse Gr-1+ neutrophils **(****FIG. 5c****,** bottom row) 18 hours after exposure to IRI.

**Anti-C5 mAb Attenuates T cell-mediated Neointimal and Thrombotic AV Lesions Following IRI.** In a final set of experiments, we assessed the effect of anti-C5 antibody or control antibody on the development of AV lesions in arteries subjected to IRI and then retransplanted into hosts that had been previously given human PBMC allogeneic to the artery donor (Fig 6a). Antibodies were introduced in the first host just prior to graft harvest, and treatments with 12B4 or BB5.1 mAb continued for an additional two weeks in the second host prior to graft harvesting and analysis. By immunofluorescence microscopy, coronary grafts treated with BB5.1 anti-C5 mAb showed significant reduction in MAC (C5b-9) formation **(****FIG. 6b****,** top row), NIK expression (Fig 6b, second row), and an ~50% decrease in mural infiltrates of alloimmune CD45RO+ T cells **(****FIG. 6b****,** third row). Interestingly, in hosts treated with either 12B4 or BB5.1 mAb, we observed significantly greater numbers of CD4+ T cells in the intima **(****FIG. 6b****,** bottom row, left graph) and significantly greater numbers of CD8⁺ T cells in the media (right graph). While significantly reducing overall numbers of both CD4+ and CD8⁺ T cells compared to hosts treated with 12B4 mAb (asterisks and double asterisks), BB5.1 mAb did not significantly alter the ratio of CD4+ T cells vs CD8⁺ T cells in the intima (p=0.76) or media (p=0.21), indicating that anti-C5 mAb reduced infiltration by CD4+ and CD8⁺ T cells to an equal extent. The changes above were associated with significantly decreased neointimal area without significant change in luminal area in BB5.1 mAb-treated hosts compared to controls, suggesting early outward remodeling of the allograft vessel during AV lesion formation **(****FIG. 6c****).** These data show that terminal complement blockade with anti-C5 antibody attenuated the formation of neointimal AV lesions following IRI.

Three out of eight hosts receiving IRI-treated grafts and treated with 12B4 mAb became moribund and developed bilateral hindlimb ischemia during the experimental protocol and were thus prematurely sacrificed at days 4, 7, and 10 post-transplant. Necropsy of harvested grafts revealed the presence of flow-limiting thromboses in all three hosts **(****FIG. 6d****,** top row) which were confirmed with fibrinogen staining **(****FIG. 6d****,** top row). Thrombosis was not due to a secondary effect of 12B4 mAb treatment as hosts implanted with grafts not exposed to hypoxia and treated with 12B4 mAb did not develop similar lesions **(****FIG. 1d****).** Remarkably, all BB5.1-treated hosts were spared from the development of thrombotic lesions during the treatment period (0/8, **FIG. 6d****,** bottom row). We conclude that anti-C5 antibody prevents both complement-mediated neointimal and thrombotic AV lesions induced by IRI.

### DISCUSSION

We demonstrate a positive effect of anti-C5 therapy on experimental AV lesions which develop following exposure to alloantibody or IRI. Repeated administration of anti-C5 blocked terminal but not early complement activation in human coronary artery tissues, resulting in attenuated activation of non-canonical NF-κB signaling and decreased formation of both stenotic and thrombotic AV lesions in vivo. Our study demonstrates a causal connection between terminal complement activation with non-canonical NF-κB activation and IRI. Hosts treated with anti-C5 mAb showed attenuated NIK expression and AV lesions compared to controls **(****FIG. 6****).** In comparison with DSA, IRI induced MAC assembly throughout the vessel wall in a broader area of distribution including endothelial cells (EC) in the intima and smooth muscle cells (SMC) in the media. Whether MAC and/or non-canonical NF-κB elicits differential functional effects in these cell types is unknown and is especially relevant considering mechanisms of immunoprivilege in the media relative to the intima (Tellides G, Pober JS. Circ. Res 2015; 116: 312-322) and also considering the observed differential spatial localizations of infiltrating CD4+ and CD8+ T cells following IRI **(****FIG. 6c****).**

In addition to the neointimal expansion, a more recently appreciated form of AV is characterized by activation of the coagulation cascade (Atkinson C et al. J Immunol 2010; 185: 7007-7013) resulting in occlusive thromboses. Tedesco F, Pausa M et al. J Exp Med. 1997; 185: 1619-1627; Jiang X et al., J Mol Med. 2014; 92: 797-810. Thrombotic AV lesions affecting microvessels (Jiang X et al., J Mol Med. 2014; 92: 797-810; Labarrere CA et al. J Heart Lung Transplant. 2006; 25: 1213-1222) and large-caliber vessels (Fishbein GA, Fishbein MC. Hum Immunol. 2012; 73:1213-1217) are associated with worsened long-term graft outcomes. Torres SA et al. Methodist Debakey Cardiovasc J. 2012; 8: 46-48. Unexpectedly, we observed that several of our vessels subjected to IRI and then transplanted into animals with circulating human T cells developed thrombosis **(****FIG. 6d****).** Anti-C5 mAb also prevented thrombotic complications as a result of transplant-associated IRI.

This study used human arteries, alloantibodies and PBMCs to model CAV, a disorder which is poorly modeled in rodents. Pober JS et al. Arterioscler Thromb Vase Biol. 2014; 34: 1609-1614. However, the complement components and the coagulation factors that are activated are of mouse origin.

### SEQUENCE SUMMARY

| |
|---|
| **SEQ ID NO:1** |
| GYIFSNYWIQ |
| **SEQ ID NO:2** |
| EILPGSGSTEYTENFKD |
| **SEQ ID NO:3** |
| YFFGSSPNWYFDV |
| **SEQ ID NO:4** |
| GASENIYGALN |
| **SEQ ID NO:5** |
| GATNLAD |
| **SEQ ID NO:6** |
| QNVLNTPLT |
| **SEQ ID NO:7** |
| |
| **SEQ ID NO:8** |
| |
| **SEQ ID NO:9** |
| ASTKGPSVFPLAPCSRSTSESTAALGCLVKDYFPEPVTVSWNSGALTSGVH TFPAVLQSSGLYSLSSVVTVPSSNFGTQTYTCNVDHKPSNTKVDKTVERKC CVECPPCPAPPVAGPSVFLFPPKPKDTLMISRTPEVTCVVVDVSQEDPEVQF NWYVDGVEVHNAKTKPREEQFNSTYRVVSVLTVLHQDWLNGKEYKCKV SNKGLPSSIEKTISKAKGQPREPQVYTLPPSQEEMTKNQVSLTCLVKGFYPS DIAVEWESNGQPENNYKTTPPVLDSDGSFFLYSRLTVDKSRWQEGNVFSCS VMHEALHNHYTQKSLSLSLGK |
| **SEQ ID NO:10** |
| QVQLVQSGAEVKKPGASVKVSCKASGYIFSNYWIQWVRQAPGQGLEWM GEILPGSGSTEYTENFKDRVTMTRDTSTSTVYMELSSLRSEDTAVYYCAR YFFGSSPNWYFDVWGQGTLVTVSSASTKGPSVFPLAPCSRSTSESTAALGCL VKDYFPEPVTVSWNSGALTSGVHTFPAVLQSSGLYSLSSVVTVPSSNFGTQTYT CNVDHKPSNTKVDKTVERKCCVECPPCPAPPVAGPSVFLFPPKPKDTLMISR TPEVTCVVVDVSQEDPEVQFNWYVDGVEVHNAKTKPREEQFNSTYRVVSVLT VLHQDWLNGKEYKCKVSNKGLPSSIEKTISKAKGQPREPQVYTLPPSQEEMT KNQVSLTCLVKGFYPSDIAVEWESNGQPENNYKTTPPVLDSDGSFFLYSRLTV DKSRWQEGNVFSCSVMHEALHNHYTQKSLSLSLGK |
| **SEQ ID NO:11** |
| |
| **SEQ ID NO:12** |
| |
| **SEQ ID NO:13** |
| ASTKGPSVFPLAPCSRSTSESTAALGCLVKDYFPEPVTVSWNSGALTSGV HTFPAVLQSSGLYSLSSVVTVPSSNFGTQTYTCNVDHKPSNTKVDKTVER KCCVECPPCPAPPVAGPSVFLFPPKPKDTLMISRTPEVTCVVVDVSQEDPE VQFNWYVDGVEVHNAKTKPREEQFNSTYRVVSVLTVLHQDWLNGKEYK CKVSNKGLPSSIEKTISKAKGQPREPQVYTLPPSQEEMTKNQVSLTCLVKG FYPSDIAVEWESNGQPENNYKTTPPVLDSDGSFFLYSRLTVDKSRWQEGN VFSCSVLHEALHSHYTQKSLSLSLGK |
| **SEQ ID NO:14** |
| QVQLVQSGAEVKKPGASVKVSCKASGHIFSNYWIQWVRQAPGQGLEWM GEILPGSGHTEYTENFKDRVTMTRDTSTSTVYMELSSLRSEDTAVYYCAR YFFGSSPNWYFDVWGQGTLVTVSS ASTKGPSVFPLAPCSRSTSESTAALGCL VKDYFPEPVTVSWNSGALTSGVHTFPAVLQSSGLYSLSSVVTVPSSNFGTQTYT CNVDHKPSNTKVDKTVERKCCVECPPCPAPPVAGPSVFLFPPKPKDTLMISR TPEVTCVVVDVSQEDPEVQFNWYVDGVEVHNAKTKPREEQFNSTYRVVSVLT VLHQDWLNGKEYKCKVSNKGLPSSIEKTISKAKGQPREPQVYTLPPSQEEMT KNQVSLTCLVKGFYPSDIAVEWESNGQPENNYKTTPPVLDSDGSFFLYSRLTV DKSRWQEGNVFSCSVLHEALHSHYTQKSLSLSLGK |
| **SEQ ID NO:15** |
| ASTKGPSVFPLAPCSRSTSESTAALGCLVKDYFPEPVTVSWNSGALTSGVH TFPAVLQSSGLYSLSSVVTVTSSNFGTQTYTCNVDHKPSNTKVDKTVERKC CVECPPCPAPPVAGPSVFLFPPKPKDTLYITREPEVTCVVVDVSHEDPEVQF NWYVDGMEVHNAKTKPREEQFNSTFRVVSVLTVVHQDWLNGKEYKCKV SNKGLPAPIEKTISKTKGQPREPQVYTLPPSREEMTKNQVSLTCLVKGFYP SDIAVEWESNGQPENNYKTTPPMLDSDGSFFLYSKLTVDKSRWQQGNVF SCSVMHEALHNHYTQKSLSLSPGK |
| **SEQ ID NO:16** |
| QVQLVQSGAEVKKPGASVKVSCKASGYIFSNYWIQWVRQAPGQGLEWM GEILPGSGSTEYTENFKDRVTMTRDTSTSTVYMELSSLRSEDTAVYYCAR YFFGSSPNWYFDVWGQGTLVTVSSASTKGPSVFPLAPCSRSTSESTAALG CLVKDYFPEPVTVSWNSGALTSGVHTFPAVLQSSGLYSLSSVVTVTSSNF GTQTYTCNVDHKPSNTKVDKTVERKCCVECPPCPAPPVAGPSVFLFPPKP KDTLYITREPEVTCVVVDVSHEDPEVQFNWYVDGMEVHNAKTKPREEQ FNSTFRVVSVLTVVHQDWLNGKEYKCKVSNKGLPAPIEKTISKTKGQPRE PQVYTLPPSREEMTKNQVSLTCLVKGFYPSDIAVEWESNGQPENNYKTTP PMLDSDGSFFLYSKLTVDKSRWQQGNVFSCSVMHEALHNHYTQKSLSLS PGK |
| **SEQ ID NO:17** |
| GASENIYHALN |
| **SEQ ID NO:18** |
| EILPGSGHTEYTENFKD |
| **SEQ ID NO:19** |
| GHIFSNYWIQ |
| **SEQ ID NO:20** |
| QVQLVQSGAEVKKPGASVKVSCKASGHIFSNYWIQWVRQAPGQGLEW MGEILPGSGHTEYTENFKDRVTMTRDTSTSTVYMELSSLRSEDTAVYYC ARYFFGSSPNWYFDVWGQGTLVTVSSASTKGPSVFPLAPCSRSTSESTAALG CLVKDYFPEPVTVSWNSGALTSGVHTFPAVLQSSGLYSLSSVVTVPSSNFGTQT YTCNVDHKPSNTKVDKTVERKCCVECPPCPAPPVAGPSVFLFPPKPKDTLMIS RTPEVTCVVVDVSQEDPEVQFNWYVDGVEVHNAKTKPREEQFNSTYRVVSVL TVLHQDWLNGKEYKCKVSNKGLPSSIEKTISKAKGQPREPQVYTLPPSQEEMT KNQVSLTCLVKGFYPSDIAVEWESNGQPENNYKTTPPVLDSDGSFFLYSRLTV DKSRWQEGNVFSCSVMHEALHNHYTQKSLSLSLGK |
| **SEQ ID NO:21** |
| SYAIS |
| **SEQ ID NO:22** |
| GIGPFFGTANYAQKFQG |
| **SEQ ID NO:23** |
| DTPYFDY |
| **SEQ ID NO:24** |
| SGDSIPNYYVY |
| **SEQ ID NO:25** |
| DDSNRPS |
| **SEQ ID NO:26** |
| QSFDSSLNAEV |
| **SEQ ID NO:27** |
| QVQLVQSGAEVKKPGSSVKVSCKASGGTFSSYAISVWRQAPGQGLEWMGGIGPFFGTANY AQKFQGRVTITADESTSTAYMELSSLRSEDTAVYYCARDTPYFD YWGQGTLVTVSS |
| **SEQ ID NO:28** |
| DIELTQPPSVSVAPGQTARISCSGDSIPNYYVYWYQQKPGQAPVLVIYDDSNRPSGIPERFSGSN SGNTATLTISGTQAEDEADYYCQSFDSSLNAEVFGGGTK LTVL |
| **SEQ ID NO:29** |
| NYIS |
| **SEQ ID NO:30** |
| IIDPDDSYTEYSPSFQG |
| **SEQ ID NO:31** |
| YEYGGFDI |
| **SEQ ID NO:32** |
| SGDNIGNSYVH |
| **SEQ ID NO:33** |
| KDNDRPS |
| **SEQ ID NO:34** |
| GTYDIESYV |
| **SEQ ID NO:35** |
| EVQLVQSGAEVKKPGESLKISCKGSGYSFTNYISWVRQMPGKGLEWMGIIDPDDSYTEYSPSFQ GQVTI SADKSISTAYLQWSSLKASDTAMYYCARYEYGGFDI WGQGTLVTVSS |
| **SEQ ID NO:36** |
| SYELTQPPSVSVAPGQTARISCSGDNIGNSYVHWYQQKPGQAPVLVIYKDNDRPSGIPERFSGS NSGNTATLTISGTQAEDEADYYCGTYDIESYVFGGGTKLTV L |

## Claims

1. An anti-C5 antibody, or antigen-binding fragment thereof, which binds to C5 and inhibits the cleavage of C5 into fragments C5a and C5b for use in a method of treating or preventing a T-cell mediated allograft vasculopathy lesion in a mammalian recipient of a transplant of an allograft from a donor, wherein the method comprises administering the anti-C5 antibody, or antigen-binding fragment thereof, to the recipient.

2. The anti-C5 antibody, or antigen-binding fragment thereof, for use according to claim 1, wherein the method further comprises a step of pre-treating the allograft with the anti-C5 antibody, or antigen-binding fragment thereof, prior to transplanting the allograft.

3. The anti-C5 antibody, or antigen-binding fragment thereof, for use according to claim 1 or claim 2, wherein the method further comprises administering to the recipient an anti-meningococcal vaccine and/or antibiotics prior to administering the anti-C5 antibody, or antigen-binding fragment thereof.

4. The anti-C5 antibody, or antigen-binding fragment thereof, for use according to any one of the preceding claims, wherein the method comprises a step of administering to the recipient an immunosuppressive therapy, wherein the immunosuppressive therapy is selected from the group consisting of corticosteroids, azathioprine (AZA), mycophenolate mofetil (MMF), methotrexate, tacrolimus, cyclosporine or cyclophosphamide either in combination or as a mono-therapy.

5. The anti-C5 antibody, or antigen-binding fragment thereof, for use according to any one of the preceding claims, wherein the anti-C5 antibody, or antigen-binding fragment thereof, is administered intravenously.

6. The anti-C5 antibody, or antigen-binding fragment thereof, for use according to any one of the preceding claims, wherein the mammalian transplant recipient is a human recipient.

7. The anti-C5 antibody, or antigen-binding fragment thereof, for use according to any one of claims 1-6, wherein the anti-C5 antibody is eculizumab.

8. The anti-C5 antibody, or antigen-binding fragment thereof, for use according to any one of claims 1-6, wherein the anti-C5 antibody is an antibody comprising heavy and light chains having the sequences shown in SEQ ID NOs: 14 and 11, respectively.

9. The anti-C5 antibody, or antigen-binding fragment thereof, for use according to any of claims 1-6, wherein the anti-C5 antibody is an antibody comprising heavy and light chains having the sequences shown in SEQ ID NOs: 20 and 11, respectively.

10. The anti-C5 antibody, or antigen-binding fragment thereof, for use according to any one of claims 1-6, wherein the anti-C5 antibody, or antigen-binding fragment thereof, comprises one of the following:
i. CDR1, CDR2, and CDR3 heavy chain sequences as set forth in SEQ ID NOs: 1, 2, and 3, respectively, and CDR1, CDR2, and CDR3 light chain sequences as set forth in SEQ ID NOs: 4, 5, and 6, respectively;
ii. CDR1, CDR2, and CDR3 heavy chain sequences as set forth in SEQ ID NOs: 19, 18, and 3, respectively, and CDR1, CDR2, and CDR3 light chain sequences as set forth in SEQ ID NOs: 4, 5, and 6, respectively;
iii. CDR1, CDR2, and CDR3 heavy chain sequences as set forth in SEQ ID NOs: 21, 22, and 23, respectively, and CDR1, CDR2, and CDR3 light chain sequences as set forth in SEQ ID NOs: 24, 25, and 26, respectively; and
iv. CDR1, CDR2, and CDR3 heavy chain sequences as set forth in SEQ ID NOs: 29, 30, and 31, respectively, and CDR1, CDR2, and CDR3 light chain sequences as set forth in SEQ ID NOs: 32, 33, and 34, respectively.

11. The anti-C5 antibody, or antigen-binding fragment thereof, for use according to any one of claims 1-6, wherein the anti-C5 antibody, or antigen-binding fragment thereof, comprises one of the following:
i. the V_{H} domain having the sequence set forth in SEQ ID NO: 7, and the V_{L} domain having the sequence set forth in SEQ ID NO: 8, respectively;
ii. the V_{H} domain having the sequence set forth in SEQ ID NO: 12, and the V_{L} domain having the sequence set forth in SEQ ID NO: 8, respectively;
iii. the V_{H} domain having the sequence set forth in SEQ ID NO: 27, and the V_{L} domain having the sequence set forth in SEQ ID NO: 28; and
iv. the V_{H} domain having the sequence set forth in SEQ ID NO: 35, and the V_{L} domain having the sequence set forth in SEQ ID NO: 36, respectively.

12. The anti-C5 antibody, or antigen-binding fragment thereof, for use according to any one of claims 1-6, wherein the anti-C5 antibody, or antigen-binding fragment thereof, comprises a heavy chain constant region having the amino acid sequences set forth in SEQ ID NO: 9.

13. The anti-C5 antibody, or antigen-binding fragment thereof, for use according to any one of claims 1-6, wherein the anti-C5 antibody, or antigen-binding fragment thereof, comprises the entire heavy chain and light chains having the amino acid sequences set forth in SEQ ID NO: 10 and SEQ ID NO: 11, respectively.

14. The anti-C5 antibody, or antigen-binding fragment thereof, for use according to any one of claims 1-6, wherein the anti-C5 antibody, or antigen-binding fragment thereof, comprises a heavy chain constant region having the amino acid sequences set forth in SEQ ID NO 13.

15. The anti-C5 antibody, or antigen-binding fragment thereof, for use according to any one of the preceding claims, wherein the method:
(a) reduces the likelihood of an ischemia reperfusion (IR) injury to the allograft;
(b) reduces the likelihood of a donor specific antibody (DSA) induced allograft injury;
(c) reduces the likelihood of thrombotic complications of transplant-associated IR injury;
(d) reduces the likelihood of IR injury induced membrane attack complex assembly in microvessels and large-caliber vessels of the allograft;
(e) reduces the likelihood of IR induced activation of non-canonical NF-κB signaling in the allograft;
(f) reduces the likelihood of IR injury induced NIK expression;
(g) reduces the likelihood of forming a diffusely expanded neointima made up of smooth muscle-like cells along with sub-endothelial infiltrates of T cells and macrophages in a vessel of the allograft; and/or
(h) reduces the likelihood of forming an occlusive thromboses in a vascular vessel of the allograft.

## Patentansprüche

1. Anti-C5-Antikörper oder antigenbindendes Fragment davon, der/das an C5 bindet und die Spaltung von C5 in die Fragmente C5a und C5b hemmt, für die Verwendung in einem Verfahren zum Behandeln oder Vorbeugen einer T-Zell-vermittelten Allotransplantat-Vaskulopathie-Läsion in einem Säugetierempfänger eines Transplantats eines Allotransplantats von einem Spender, wobei das Verfahren das Verabreichen des Anti-C5-Antikörpers oder des antigenbindenden Fragments davon an den Empfänger umfasst.

2. Anti-C5-Antikörper oder antigenbindendes Fragment davon für die Verwendung nach Anspruch 1, wobei das Verfahren ferner einen Schritt des Vorbehandelns des Allotransplantats mit dem Anti-C5-Antikörper oder dem antigenbindenden Fragment davon vor der Transplantation des Allotransplantats umfasst.

3. Anti-C5-Antikörper oder antigenbindendes Fragment davon für die Verwendung nach Anspruch 1 oder 2, wobei das Verfahren ferner das Verabreichen eines Anti-Meningokokken-Impfstoffs und/oder von Antibiotika an den Empfänger vor der Verabreichung des Anti-C5-Antikörpers oder des antigenbindenden Fragments davon umfasst.

4. Anti-C5-Antikörper oder antigenbindendes Fragment davon für die Verwendung nach einem der vorhergehenden Ansprüche, wobei das Verfahren einen Schritt des Verabreichens einer immunsuppressiven Therapie an den Empfänger umfasst, wobei die immunsuppressive Therapie aus der Gruppe ausgewählt ist, bestehend aus Kortikosteroiden, Azathioprin (AZA), Mycophenolatmofetil (MMF), Methotrexat, Tacrolimus, Cyclosporin oder Cyclophosphamid entweder in Kombination oder als Monotherapie.

5. Anti-C5-Antikörper oder antigenbindendes Fragment davon für die Verwendung nach einem der vorhergehenden Ansprüche, wobei der Anti-C5-Antikörper oder das antigenbindende Fragment davon intravenös verabreicht wird.

6. Anti-C5-Antikörper oder antigenbindendes Fragment davon für die Verwendung nach einem der vorhergehenden Ansprüche, wobei der Säugetier-Transplantatempfänger ein menschlicher Empfänger ist.

7. Anti-C5-Antikörper oder antigenbindendes Fragment davon für die Verwendung nach einem der Ansprüche 1-6, wobei der Anti-C5-Antikörper Eculizumab ist.

8. Anti-C5-Antikörper oder antigenbindendes Fragment davon für die Verwendung nach einem der Ansprüche 1-6, wobei der Anti-C5-Antikörper ein Antikörper ist, der schwere und leichte Ketten mit den in SEQ ID NO: 14 beziehungsweise 11 gezeigten Sequenzen umfasst.

9. Anti-C5-Antikörper oder antigenbindendes Fragment davon für die Verwendung nach einem der Ansprüche 1-6, wobei der Anti-C5-Antikörper ein Antikörper ist, der schwere und leichte Ketten mit den in SEQ ID NO: 20 beziehungsweise 11 gezeigten Sequenzen umfasst.

10. Anti-C5-Antikörper oder antigenbindendes Fragment davon für die Verwendung nach einem der Ansprüche 1-6, wobei der Anti-C5-Antikörper oder das antigenbindende Fragment davon eines der Folgenden umfasst:
i. CDR1-, CDR2- und CDR3-Sequenzen der schweren Kette, wie in SEQ ID NO: 1, 2 beziehungsweise 3 dargelegt, und CDR1-, CDR2- und CDR3-Sequenzen der leichten Kette, wie in SEQ ID NO: 4, 5 beziehungsweise 6 dargelegt;
ii. CDR1-, CDR2- und CDR3-Sequenzen der schweren Kette, wie in SEQ ID NO: 19, 18 beziehungsweise 3 dargelegt, und CDR1-, CDR2- und CDR3-Sequenzen der leichten Kette, wie in SEQ ID NO: 4, 5 beziehungsweise 6 dargelegt;
iii. CDR1-, CDR2- und CDR3-Sequenzen der schweren Kette, wie in SEQ ID NO: 21, 22 beziehungsweise 23 dargelegt, und CDR1-, CDR2- und CDR3-Sequenzen der leichten Kette, wie in SEQ ID NO: 24, 25 beziehungsweise 26 dargelegt; und
iv. CDR1-, CDR2- und CDR3-Sequenzen der schweren Kette, wie in SEQ ID NO: 29, 30 beziehungsweise 31 dargelegt, und CDR1-, CDR2- und CDR3-Sequenzen der leichten Kette, wie in SEQ ID NO: 32, 33 beziehungsweise 34 dargelegt.

11. Anti-C5-Antikörper oder antigenbindendes Fragment davon für die Verwendung nach einem der Ansprüche 1-6, wobei der Anti-C5-Antikörper oder das antigenbindende Fragment davon eines der Folgenden umfasst
i. die V_{H}-Domäne mit der in SEQ ID NO: 7 dargelegten Sequenz beziehungsweise die V_{L}-Domäne mit der in SEQ ID NO: 8 dargelegten Sequenz;
ii. die V_{H}-Domäne mit der in SEQ ID NO: 12 dargelegten Sequenz beziehungsweise die V_{L}-Domäne mit der in SEQ ID NO: 8 dargelegten Sequenz;
iii. die V_{H}-Domäne mit der in SEQ ID NO: 27 dargelegten Sequenz beziehungsweise die V_{L}-Domäne mit der in SEQ ID NO: 28 dargelegten Sequenz;
iv. die V_{H}-Domäne mit der in SEQ ID NO: 35 dargelegten Sequenz beziehungsweise die V_{L}-Domäne mit der in SEQ ID NO: 36 dargelegten Sequenz.

12. Anti-C5-Antikörper oder antigenbindendes Fragment davon für die Verwendung nach einem der Ansprüche 1-6, wobei der Anti-C5-Antikörper oder das antigenbindende Fragment davon eine konstante Region der schweren Kette mit den in SEQ ID NO: 9 dargelegten Aminosäuresequenzen umfasst.

13. Anti-C5-Antikörper oder antigenbindendes Fragment davon für die Verwendung nach einem der Ansprüche 1-6, wobei der Anti-C5-Antikörper oder das antigenbindende Fragment davon die gesamte schwere Kette und leichte Kette mit den in SEQ ID NO: 10 beziehungsweise SEQ ID NO: 11 dargestellten Aminosäuresequenzen umfasst.

14. Anti-C5-Antikörper oder antigenbindendes Fragment davon für die Verwendung nach einem der Ansprüche 1-6, wobei der Anti-C5-Antikörper oder das antigenbindende Fragment davon eine konstante Region der schweren Kette mit den in SEQ ID NO: 13 dargestellten Aminosäuresequenzen umfasst.

15. Anti-C5-Antikörper oder antigenbindendes Fragment davon für die Verwendung nach einem der vorhergehenden Ansprüche, wobei das Verfahren:
(a) die Wahrscheinlichkeit einer Ischämie-Reperfusionsverletzung (IR-Verletzung) des Allotransplantats reduziert;
(b) die Wahrscheinlichkeit einer durch spenderspezifische Antikörper (DSA) induzierten Allotransplantatverletzung reduziert;
(c) die Wahrscheinlichkeit thrombotischer Komplikationen einer transplantatassoziierten IR-Verletzung reduziert;
(d) die Wahrscheinlichkeit einer Membranangriffskomplexzusammensetzung in den Mikrogefäßen und großkalibrigen Gefäßen des Allotransplantats, die durch die IR-Verletzung induziert wird, reduziert;
(e) die Wahrscheinlichkeit einer IR-induzierten Aktivierung der nicht-kanonischen NF-xB-Signalisierung in dem Allotransplantat reduziert;
(f) die Wahrscheinlichkeit einer durch IR-Verletzung induzierten NIK-Expression reduziert;
(g) die Wahrscheinlichkeit des Ausbildens einer diffus ausgedehnten Neointima, die aus glatten muskelähnlichen Zellen zusammen mit subendothelialen Infiltraten von T-Zellen und Makrophagen in einem Gefäß des Allotransplantats besteht, reduziert; und/oder
(h) die Wahrscheinlichkeit des Ausbildens einer okklusiven Thrombose in einem Gefäß des Allotransplantats reduziert.

## Revendications

1. Anticorps anti-C5, ou fragment de liaison à l'antigène de celui-ci, qui se lie à C5 et empêche le clivage de C5 en fragments C5a et C5b pour l'utilisation dans une méthode de traitement ou de prévention d'une lésion vasculopathique d'allogreffon médiée par cellules T chez un receveur mammifère d'un transplant d'un allogreffon provenant d'un donneur, dans lequel la méthode comprend l'administration de l'anticorps anti-C5, ou du fragment de liaison à l'antigène de celui-ci, au receveur.

2. Anticorps anti-C5, ou fragment de liaison à l'antigène de celui-ci, pour l'utilisation selon la revendication 1, dans lequel la méthode comprend en outre une étape du prétraitement de l'allogreffon avec l'anticorps anti-C5, ou le fragment de liaison à l'antigène de celui-ci, avant la transplantation de l'allogreffon.

3. Anticorps anti-C5, ou fragment de liaison à l'antigène de celui-ci, pour l'utilisation selon la revendication 1 ou la revendication 2, dans lequel la méthode comprend en outre l'administration, au receveur, d'un vaccin anti-méningococcique et/ou d'antibiotique avant l'administration de l'anticorps anti-C5, ou du fragment de liaison à l'antigène de celui-ci.

4. Anticorps anti-C5, ou fragment de liaison à l'antigène de celui-ci, pour l'utilisation selon l'une quelconque des revendications précédentes, dans lequel la méthode comprend une étape de l'administration, au receveur, d'une thérapie immunosuppressive, dans lequel la thérapie immunosuppressive est sélectionnée parmi le groupe constitué de : corticostéroïdes, azathioprine (AZA), mycophénolate mofétil (MMF), méthotrexate, tacrolimus, cyclosporine ou cyclophosphamide, soit en association soit sous forme de monothérapie.

5. Anticorps anti-C5, ou fragment de liaison à l'antigène de celui-ci, pour l'utilisation selon l'une quelconque des revendications précédentes, dans lequel l'anticorps anti-C5, ou le fragment de liaison à l'antigène de celui-ci, est administré par voie intraveineuse.

6. Anticorps anti-C5, ou fragment de liaison à l'antigène de celui-ci, pour l'utilisation selon l'une quelconque des revendications précédentes, dans lequel le receveur de transplant mammifère est un receveur humain.

7. Anticorps anti-C5, ou fragment de liaison à l'antigène de celui-ci, pour l'utilisation selon l'une quelconque des revendications 1 à 6, dans lequel l'anticorps anti-C5 est éculizumab.

8. Anticorps anti-C5, ou fragment de liaison à l'antigène de celui-ci, pour l'utilisation selon l'une quelconque des revendications 1 à 6, dans lequel l'anticorps anti-C5 est un anticorps comprenant des chaînes lourde et légère ayant les séquences présentées dans SEQ ID n° : 14 et 11, respectivement.

9. Anticorps anti-C5, ou fragment de liaison à l'antigène de celui-ci, pour l'utilisation selon l'une quelconque des revendications 1 à 6, dans lequel l'anticorps anti-C5 est un anticorps comprenant des chaînes lourde et légère ayant les séquences présentées dans SEQ ID n° : 20 et 11, respectivement.

10. Anticorps anti-C5, ou fragment de liaison à l'antigène de celui-ci, pour l'utilisation selon l'une quelconque des revendications 1 à 6, dans lequel l'anticorps anti-C5, ou le fragment de liaison à l'antigène de celui-ci, comprend une de ce qui suit :
i. des séquences de chaîne lourde de CDR1, CDR2, et CDR3 telles que présentées dans SEQ ID n° : 1, 2, et 3, respectivement, et des séquences de chaîne légère de CDR1, CDR2, et CDR3 telles que présentées dans SEQ ID n° : 4, 5, et 6, respectivement ;
ii. des séquences de chaîne lourde de CDR1, CDR2, et CDR3 telles que présentées dans SEQ ID n° : 19, 18, et 3, respectivement, et des séquences de chaîne légère de CDR1, CDR2, et CDR3 telles que présentées dans SEQ ID n° : 4, 5, et 6, respectivement ;
iii. des séquences de chaîne lourde de CDR1, CDR2, et CDR3 telles que présentées dans SEQ ID n° : 21, 22, et 23, respectivement, et des séquences de chaîne légère de CDR1, CDR2, et CDR3 telles que présentées dans SEQ ID n° : 24, 25, et 26, respectivement ; et
iv. des séquences de chaîne lourde de CDR1, CDR2, et CDR3 telles que présentées dans SEQ ID n° : 29, 30, et 31, respectivement, et des séquences de chaîne légère de CDR1, CDR2, et CDR3 telles que présentées dans SEQ ID n° : 32, 33, et 34, respectivement.

11. Anticorps anti-C5, ou fragment de liaison à l'antigène de celui-ci, pour l'utilisation selon l'une quelconque des revendications 1 à 6, dans lequel l'anticorps anti-C5, ou le fragment de liaison à l'antigène de celui-ci, comprend un de ce qui suit :
i. le domaine V_{H} ayant la séquence représentée dans SEQ ID n° : 7, et le domaine V_{L} ayant la séquence représentée dans SEQ ID n° : 8, respectivement ;
ii. le domaine V_{H} ayant la séquence représentée dans SEQ ID n° : 12, et le domaine V_{L} ayant la séquence représentée dans SEQ ID n° : 8, respectivement ;
iii. le domaine V_{H} ayant la séquence représentée dans SEQ ID n° : 27, et le domaine V_{L} ayant la séquence représentée dans SEQ ID n° : 28 ; et
iv. le domaine V_{H} ayant la séquence représentée dans SEQ ID n° : 35, et le domaine V_{L} ayant la séquence représentée dans SEQ ID n° : 36, respectivement.

12. Anticorps anti-C5, ou fragment de liaison à l'antigène de celui-ci, pour l'utilisation selon l'une quelconque des revendications 1 à 6, dans lequel l'anticorps anti-C5, ou le fragment de liaison à l'antigène de celui-ci, comprend une région constante de chaîne lourde ayant les séquences d'acides aminés représentées dans SEQ ID n° : 9.

13. Anticorps anti-C5, ou fragment de liaison à l'antigène de celui-ci, pour l'utilisation selon l'une quelconque des revendications 1 à 6, dans lequel l'anticorps anti-C5, ou le fragment de liaison à l'antigène de celui-ci, comprend les chaîne lourde et chaînes légères entières ayant les séquences d'acides aminés représentées dans SEQ ID n° : 10 et SEQ ID n° : 11, respectivement.

14. Anticorps anti-C5, ou fragment de liaison à l'antigène de celui-ci, pour l'utilisation selon l'une quelconque des revendications 1 à 6, dans lequel l'anticorps anti-C5, ou le fragment de liaison à l'antigène de celui-ci, comprend une région constante de chaîne lourde ayant les séquences d'acides aminés représentées dans SEQ ID n° : 13.

15. Anticorps anti-C5, ou fragment de liaison à l'antigène de celui-ci, pour l'utilisation selon l'une quelconque des revendications précédentes, dans lequel la méthode :
(a) réduit la probabilité d'une lésion d'ischémie reperfusion (IR) sur l'allogreffon ;
(b) réduit la probabilité d'une lésion d'allogreffon induite par anticorps spécifique de donneur (DSA),
(c) réduit la probabilité de complications thrombotiques de lésion IR associée à transplant,
(d) réduit la probabilité d'ensemble complexe d'attaque membranaire induit par lésion IR dans des micro-vaisseaux et des vaisseaux de gros calibre de l'allogreffon ;
(e) réduit la probabilité d'activation induite par IR de signalisation NF-κB non canonique dans l'allogreffon ;
(f) réduit la probabilité d'expression NIK induite par lésion IR ;
(g) réduit la probabilité de formation d'une néo-intima à croissance diffuse composée de cellules de type muscle lisse conjointement avec des infiltrats sous-endothéliaux de cellules T et de macrophages dans un vaisseau de l'allogreffon ; et/ou
(h) réduit la probabilité de formation d'une thrombose occlusive dans un vaisseau vasculaire de l'allogreffon.
